(19) Europäisches Patentamt

European Patent Office

Office européen des brevets

(11) **EP 1 369 493 B1**

(12) **EUROPÄISCHE PATENTSCHRIFT**

(45) Veröffentlichungstag und Bekanntmachung des Hinweises auf die Patenterteilung:
**13.09.2006 Patentblatt 2006/37**

(51) Int Cl.:
*C12Q 1/68* (2006.01)

(21) Anmeldenummer: **02090203.7**

(22) Anmeldetag: **05.06.2002**

(54) **Verfahren zur quantitiven Bestimmung des Methylierungsgrades von Cytosinen in CpG-Positionen**

Quantitative determination method for the degree of methylation of cytosines in CpG positions

Procédé pour la détermination quantitative du degré de methylation de cytosines en positions CpG

(84) Benannte Vertragsstaaten:
**AT BE CH CY DE DK ES FI FR GB GR IE IT LI LU MC NL PT SE TR**

(43) Veröffentlichungstag der Anmeldung:
**10.12.2003 Patentblatt 2003/50**

(73) Patentinhaber: **Epigenomics AG**
**10178 Berlin (DE)**

(72) Erfinder:
• **Piepenbrock, Christian**
**10115 Berlin (DE)**
• **Lewin, Joern**
**10787 Berlin (DE)**
• **Schmitt, Armin Dr.**
**12203 Berlin (DE)**

(74) Vertreter: **Schubert, Klemens**
**Neue Promenade 5**
**10178 Berlin-Mitte (DE)**

(56) Entgegenhaltungen:
**WO-A-98/56952**

• **PAUL C L ET AL: "Cytosine methylation: quantitation by automated genomic sequencing and GENESCAN analysis" BIOTECHNIQUES, EATON PUBLISHING, NATICK, US, Bd. 21, Nr. 1, Juli 1996 (1996-07), Seiten 126-133, XP002143107 ISSN: 0736-6205**
• **EL-MAARRI OSMAN ET AL: "A rapid, quantitative, non-radioactive bisulfite-SNuPE- IP RP HPLC assay for methylation analysis at specific CpG sites." NUCLEIC ACIDS RESEARCH. ENGLAND 15 MAR 2002, Bd. 30, Nr. 6, 15. März 2002 (2002-03-15), Seite e25 XP002216942 ISSN: 1362-4962**

**Beschreibung**

Die Erfindung betrifft ein Verfahren zur Bestimmung des Methylierungsgrades eines zu untersuchenden Cytosins im Genom wenigstens einer Zelle oder wenigstens eines Organismus.

**Stand der Technik**

5-Methylcytosin ist die häufigste kovalent modifizierte Base in der DNA eukaryotischer Zellen. Sie spielt beispielsweise eine Rolle in der Regulation der Transkription, beim genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht per se durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Es sind mehrere Verfahren bekannt, die diese Probleme lösen. Meist wird eine chemische Reaktion oder enzymatische Behandlung der genomischen DNA durchgeführt, infolge derer sich die Cytosin- von den Methylcytosin-Basen unterscheiden lassen. Eine gängige Methode ist die Umsetzung von genomischer DNA mit Bisulfit, die nach alkalischer Hydrolyse in zwei Schritten zu einer Umwandlung der Cytosin-Basen in Uracil führt (Shapiro, R., Cohen, B. und Servis, R. (1970) Nature 227, 1047). 5-Methylcytosin bleibt unter diesen Bedingungen unverändert. Die Umwandlung von Cytosin in Uracil führt zu einer Veränderung der Basensequenz, aus der sich durch Sequenzierung nun die ursprünglichen 5-Methylcytosine ermitteln lassen (nur diese liefern noch eine Bande in der C-Spur).

Eine Übersicht über die weiteren bekannten Möglichkeiten, 5-Methylcytosine nachzuweisen, ist mitsamt der dazugehörigen Literatur dem folgenden Übersichtsartikel zu entnehmen: Rein, T., DePamphilis, M. L. und Zorbas, H. (1998) Nucleic Acids Res. 26, 2255.

Die Bestimmung einer Basenabfolge anhand von Zugabe geeigneter Oligonukleotide, die sich an die DNA binden und als Primer-Moleküle fungieren, welche dann mit Hilfe einer DNA-Polymerase und ausreichend dNTPs sowie zum Kettenabbruch führenden ddNTPs verlängert werden, ist ebenfalls Stand der Technik. Diese Methode wird im Folgenden Kettenabbruch- oder auch Sanger-Sequenzierungs-Methode genannt. Die meisten modernen Sequenzierungsmethoden, die eine Automatisierung der Sequenzierung erlauben, beruhen auf einer Kombination dieses Konzeptes mit der Polymerase-Kettenreaktion (PCR) und computerunterstützter Auswertungsprogramme.

Die Amplifikation von DNA mittels PCR ist Stand der Technik. Ebenso ist die automatisierte Sequenzanalyse Stand der Technik. Hauptsächlich wird für die Automatisierung die Sanger-Sequenzierungsmethode verwandt und zwar werden grundsätzlich vier Reaktionen unterschieden, in denen zu dem DNA-Templat und den vier dNTPs jeweils eine der vier Basen als ddNTP zugefügt wird. Dies führt dann zum Abbruch der Polymerase-Reaktion an jeweils dieser Basenart. Setzt man vier Reaktionsansätze an und verwendet per Ansatz spezifisch fluoreszenz-markierte Oligonukleotidprimer, lässt sich mit geeigneten Detektoren auch nach Vermischen der beendeten Reaktionsansätze noch bestimmen, aus welchem Ansatz das Molekül stammt. Damit ist es möglich die Auftrennung dieses Gemisches nach Größe in einer Spur durchzuführen.

Um die relativ aufwendige und teure Fluoreszenz-Markierung der Oligonukleotidprimer zu umgehen, lassen sich auch die Abbruch-dideoxy-Nukleotide mit verschiedenen Farben fluoreszenz-markieren. In diesem Fall erübrigt sich sogar die Trennung in vier Reaktionsansätze und es finden alle vier Abbruchreaktionen nebeneinander statt.

Nach Beendigung der Reaktion werden die fluoreszenzmarkierten Reaktionsprodukte durch Elektrophorese in einer Spur eines Acrylamidgels ihrer Größe nach aufgetrennt. Die an dem Detektor in der Reihenfolge ihrer Größe vorbei wandernden DNA-Bruchstücke werden von einem Laser bestrahlt, der den Fluoreszenzfarbstoff anregt und die Emission dessen spezifischer Wellenlänge induziert. Diese werden von dem Detektor aufgezeichnet und mit geeigneten Computerprogrammen in Sequenzinformation übersetzt.

Heute gängige automatisierte Verfahren liefern als Ergebnis dieser Ansammlung von Emissions-Daten ein Elektropherogramm, also eine graphische Darstellung dieser Daten, für jeden der vier Farbstoffe. Da jeder Farbstoff zu einer bestimmten Nukleinsäure gehört kann daraus die Basenabfolge in der Original-Sequenz ermittelt werden.

Einige Artikel, die den Stand der Technik was die "Bisulfit-Sequenzierung", also die Sequenzanalyse von mit Bisulfit behandelter DNA betrifft, beschreiben, sind im Folgenden genannt:

Beispielsweise wird die Technik der Bisulfit-Sequenzierung sowohl zur Methylierungsanalyse des Minimal-Promoters des Androgen-Rezeptors (Kinoshita et al., Methylation of the Androgen Receptor Minimal Promoter Silences Transcription in Human Prostate Cancer (2000) Cancer Research 60: 3623-3630) als auch zur Methylierungsanalyse des Glutathion-S-transferase-π-gens (Millar et al. (1999); Detailed methylation analysis of the glutathion-S-transferase π (GSTP1) gene in prostate cancer. Oncogene 18: 1313-1324) zur Aufklärung deren Rolle bei Prostata Krebs herangezogen. In beiden Artikeln nehmen die Autoren Bezug auf einen Artikel von Susan J. Clark et al. (High sensitivity mapping of methylated cytosines. (1994) Clark et al.; Nucleic Acids Res 22: 2990-2997), in dem die Methode der Bisulfit-Sequenzierung beschrieben wird.

Der Stand der Technik bezüglich der quantitativen Bestimmung der Cytosin Methylierung mit Hilfe von Sequenzierungsmethoden ist hauptsächlich einem Artikel von Paul und Clark zu entnehmen, in dem eine Methode des Sequenzierens unter Verwendung von unterschiedlich markierten Thymin und Cytosinbasen beschrieben wird (Paul C.L. and Clark S.J. (1996) Cytosine methylation: quantitation by automated genomic sequencing and GENESCAN analysis. Biotechniques. 21(1):126-33). Die Methode ist prinzipiell dazu geeignet quantitative Methylierungsgradanalysen vorzunehmen. Sie löst jedoch nicht das Problem, dass durch unterschiedliche Einbauraten von Thymin und Cytosin, sowie durch die Verwendung unterschiedlicher Farbstoffe eine genaue Quantifizierung sehr schwierig ist. Dies wird durch die in der hier beschriebenen Erfindung vorgeschlagenen Normierung gelöst. Außerdem berücksichtigt die von Clark und Paul beschriebene Methode in keinerlei Form die das Ergebins wesentlich beeinflussende Effizienz der Umwandlungsreaktion, welche die unmethylierten Cytosine in Basen mit dem Hybridisierungsverhalten von Thyminen umwandelt. Genau die Berücksichtigung einer Bisulfit-Konversionsrate ist jedoch ein wesentlicher Schritt der Methode der vorliegenden Erfindung.

Beschreibung des Problems und dessen Lösung In einem Elektropherogramm, wie oben erwähnt, wird die Abfolge der Basen in der Sequenz als Kurvenverlauf dargestellt. Theoretisch entspricht jeder Peak dieses Kurvenverlaufs dem Auftauchen einer Base in der Originalsequenz. So werden vier verschiedenfarbige Graphen übereinander gelegt, um die Gesamtsequenz zu ermitteln.

Da in einer einzelnen Sequenz jede Position nur einmal besetzt sein kann und ebenso jede Position durch wenigstens eine Base vertreten sein muss, sollten im Idealfall alle Signale die gleiche Intensität haben. Die Signalintensität ist bestimmt durch die Peakgröße, genauer gesagt durch die Fläche unter dem Peak. Diese sollten gleichmäßig groß sein, solange der Einbau der Abbruch-Nukleotide zufallsverteilt und gleichmäßig ist. Kommt es hingegen an einer Position zum begünstigten Einbau eines Abbruch-Nukleotids, liegt dieses Produkt vermehrt vor und der entsprechende Peak wird größer. Die Basenabfolge jedoch bleibt davon unberührt.

Auch die unterschiedliche Intensität der verwendeten Fluoreszenzfarbstoffe bedingt unterschiedliche Signalintensitäten. Dies spielt für die qualitative Ermittlung der Sequenz selbst keine Rolle, untersucht man jedoch ein Gemisch von Sequenzen, die sich in ihrer Basenabfolge an einzelnen Positionen unterscheiden, und will man das Verhältnis jener spezifischen Sequenzen zueinander bestimmen, erhält die korrekte Ermittlung der Peakintensität entscheidende Bedeutung. Die Intensität eines Peaks spiegelt die Anzahl der Moleküle in dem DNA-Gemisch wider, die an jener Position diese spezifische Base aufweisen. Wenn zum Beispiel zwei Allele an einer Position unterschiedliche Nukleotide aufweisen, erhält man im Elektropherogramm zwei Peaks halber Intensität an der selben Stelle, also sozusagen übereinander. Dies ist zum Beispiel der Fall, wenn Punktmutationen analysiert werden, die nur auf einem Allel auftauchen.

Die Bestimmung der Peakintensität ist folglich immer dann von entscheidender Bedeutung, wenn aus einem Gemisch von ähnlichen Sequenzen der Anteil einer bestimmte Basenabfolge quantifiziert werden soll. Dies ist der Fall, wenn man den Methylierungsgrad eines oder mehrerer Cytosine bestimmen will. Unter der quantitativen Bestimmung des Methylierungsgrads eines Cytosins ist die Bestimmung des Anteils an methylierten DNA-Molekülen in einem Gemisch aus methylierten und nicht-methylierten DNA-Molekülen zu verstehen.

Durch eine geeignete Umsetzung, beispielsweise durch Bisulfit-Behandlung, lassen sich methylierte Cytosine von nicht methylierten Cytosinen aufgrund ihres nach der Umwandlung unterschiedlichen Hybridisierungsverhalten mittels PCR unterscheiden. Es ergibt sich folglich nach der PCR die Situation, ein Gemisch von Molekülen vor sich zu haben, welches sich an einzelnen Nukleotiden voneinander unterscheidet. Sind beispielsweise 20% der Moleküle an einer CpG-Position methyliert, sollte bei der Sequenzierung der mit Bisulfit behandelten DNA an jener Position im Cytosin-detektierenden Elektropherogramm ein Peak mit einer Signalintensität von 20% und im Thymindetektierenden Elektropherogramm ein Peak mit einer Signalintensität von 80% der sonst üblichen Signalintensität auftauchen. Um die Peakintensitäten zu einer akkuraten Quantifizierung heranziehen zu können, muss allerdings vorausgesetzt werden, dass die erhaltenen Signalintensitäten für alle vier Farben identisch sind. Dies ist jedoch nicht der Fall.

Es kann im Gegenteil keinesfalls angenommen werden, dass alle vier Fluoreszenzfarbstoffe mit gleicher Intensität detektiert werden, noch dass alle vier Einbauraten gleich effektiv sind. Insbesondere bei der Sequenzierung von mit Bisulfit behandelter DNA ergibt sich das Problem, dass es verhältnismäßig sehr wenige Cytosine in der modifizierten Sequenz gibt und diese Signale daher wesentlich intensiver erscheinen. Basierend auf diesem extremen Ungleichgewicht der mit verschiedenen Farben markierten Sequenzfragmente kommt es somit zu einer Überbewertung der Signalintensität der Cytosin-Markierung und verfälscht damit das Ergebnis der Sequenzanalyse.

Um eine verlässlichere Sequenzinformation zu erhalten, insbesondere, wenn man nicht nur eine qualitative Ja/Nein Antwort erhalten möchte, sondern quantitativ den Anteil einer Base an einer spezifischen Position der Sequenz ermitteln möchte, ist eine Normierung der verschiedenen Signale untereinander empfehlenswert. Handelt es sich hingegen um die Ermittlung des Anteils von Cytosinen in einer mit Bisulfit behandelten Cytosin-armen Sequenz, wird die Normierung insbesondere des Cytosin-Signals zwingend erforderlich.

Ein weiteres wesentliches Problem ergibt sich durch die oftmals nicht bis zur vollständigen Umsetzung erfolgte Umwandlung eines unmethylierten Cytosins zu Uracil. Eine Umwandlungsrate von zum Beispiel 80% hat zur Folge, dass 20% der Cytosine, die eigentlich nach der Umwandlung zu Uracil und nach PCR ein Thymin-Signal geben sollten, dieses nicht tun. Damit beschreibt aber eine im Vergleich zur durchschnittlichen Guanin-Signalintensität nur 80%-ige Signalintensität des Thymins an der Stelle eines nicht-methylierten Cytosins eine CpG-Position, in der das Cytosin zu 100% nicht methyliert ist. Ohne die Kenntnis der Umwandlungsrate, und unter der Annahme die Umwandlung wäre vollständig verlaufen, würde diese Signalintensität als zu 80% unmethylierte und zu 20% methylierte CpG-Position interpretiert werden. Somit ist die Erfassung der Umwandlungsrate zur akkuraten Quantifizierung des Methylierungsgrades eines Cytosins ebenso zwingend erforderlich wie die Normierung der Intensitäten.

Der überwiegende Teil der Fachliteratur, der sich mit "Gene Silencing", also der Herunter-Regulierung der Gentranskription, und Methylierung befasst, beruht auf qualitativen Methylierungsanalysen, die sich auf die Analyse einer oder weniger CpG-Positionen mit Hilfe von methylierungssensitiven Restriktionsenzymen beschränken. Darüberhinausgehende Analysen, die zum Beispiel die Methylierungsgrade an unterschiedlichen Allelen untersuchen, beruhen auf Sequenzierung von Klonen (Kinoshita et al., 2000; Cancer Research 60, 3623-3630): Die zu untersuchende genomische DNA wird extrahiert und mit Bisulfit behandelt. Die interessanten DNA-Bereiche werden mittels PCR amplifiziert und die erhaltenen Amplifikate subkloniert.

Soll zum Beispiel der Methylierungsstatus spezifischer Allele bestimmt werden, werden die PCR-Amplifikate der mit Bisulfit behandelten DNA subkloniert und in E.coli transformiert. Im Anschluss daran werden 10-25 einzelne Kolonien jedes Klons untersucht. Bei der Methylierungsanalyse eines Allels, in der es nur die Ergebnisse null, eins oder zwei geben kann, erhält man bereits nach Sequenzierung einer relativ geringen Anzahl an Kolonien ein aussagekräftiges Ergebnis. Generell gilt jedoch, dass je mehr Kolonien untersucht werden desto eher entspricht das Ergebnis dem tatsächlich vorliegenden Methylierungsgrad. In dem zitierten Dokument (Kinoshita et al. 2000) werden 10-25 E. coli-Kulturen herangezogen und jeweils deren Plasmid DNA isoliert. Diese 10-25 Plasmid-DNA-Proben werden alle einzeln sequenziert und anhand des Verhältnisses von methylierten und nicht methylierten Klonen der Methylierungsgrad des Allels bestimmt.

Die Mehrfachsequenzierung der DNA und vor allem auch der zusätzliche Schritt der Klonierung macht diesen Prozess kosten-, material- und arbeitsintensiv.

In den bereits erwähnten Studien (Kinoshita et al., 2000 und Millar et ai., 1999) werden auch Versuche beschrieben, den Methylierungsgrad einer CpG-Position quantitativ zu bestimmen ohne klonieren zu müssen. Die Interpretation der Daten erfolgt jedoch in beiden Fällen ohne die Effizienz der Bisulfitreaktion bestimmt zu haben und sie damit für die Berechnung des tatsächlichen Methylierungsgrades berücksichtigen zu können. Ebenso wenig wird die Verzerrung der Signalintensitäten in Betracht gezogen. Die geschilderten herkömmlichen Methoden zur Bestimmung von DNA-Methylierung an CpG-Position mittels Sequenzierung sind daher nicht nur teuer und langwierig sondern führen auch, vor allem bei komplexeren Probe-Gemischen zu potentiell falschen Ergebnissen.

Weiterhin beschreiben Osman El-Maarri et al. ("A rapid, quantitative, non-radioactive bisulfite-SnuPE-IP RP HPLC assay for methylation at specific CpG sites", Nucleic Acids Research, 2002, Vol. 30, No. 6 e25) ein quantitatives Verfahren zur Methylierungsanalyse an spezifischen CpG-Positionen unter Verwendung von PCR-Produkten von mit Bisulfit behandelter genomischer DNA. Hierbei wird die Quantifizierung der Methylierung mittels SNuPE (single nucleotide primer extension) beschrieben.

Die WO 98 56952 A beschreibt ein Verfahren zur Krebsdiagnostik, das auf einer unterschiedlichen Methylierung der DNA basiert. Hierbei wird die Verwendung von Ms-SNuPE (methylation-sensitive single nucleotide primer extension) beschrieben.

Beschreibung der Erfindung

Die geschilderten Probleme und Nachteile des Standes der Technik werden durch das erfindungsgemäße Verfahren gelöst.

Die Aufgabe wird also erfindungsgemäß gelöst durch ein Verfahren zur Bestimmung des Methylierungsgrades eines zu untersuchenden Cytosins gemäß Anspruch 1.

Dabei ist es erfindungsgemäß bevorzugt, dass man in der Sequenzanalyse des Amplifikats nur die Thymin-oder im revers-komplementären Strang nur die Adenin-Sequenz der DNA ermittelt und das Ergebnis in Form eines Elektropherogramms darstellt.

Erfindungsgemäß wird die Signalintensität $I_B$ einer Base B, wobei B entweder C, A, G oder T sein kann und damit die Basenart festlegt, deren Intensität gemeint ist, als Teil der Fläche unter dem Peak im Elektropherogramm durch folgende Formel beschrieben, wobei f(x) den Kurvenverlauf des Intensitätssignals über einer Einheit x beschreibt, welche die Zeit wiedergibt, die nach chromatographischer Trennung den Abstand zwischen zwei Basen beschreibt:

$$I_B = \int_{W1}^{W2} f(x)dx$$

wobei W1 und W2 die x-Koordinaten der dem x-Wert des Maximum $f^{max}$ am nächsten gelegenen Wendepunkte sind und W1 links und W2 rechts von diesem x-Wert liegen.

Erfindungsgemäß wird aber auch die Signalintensität $I_B$ durch folgende Formel beschrieben:

$$I_B = \int\limits_{M1}^{M2} f(x)dx$$

wobei M1 und M2 die x-Koordinaten der dem x-Wert des Maximum $f^{max}$ am nächsten gelegenen Minima sind und M1 links und M2 rechts von diesem x-Wert liegen.

Erfindungsgemäß wird die Signalintensität $I_B$ aber auch durch folgende Formel beschrieben:

$$I_B = \int\limits_{H1}^{H2} f(x)dx$$

wobei H1 und H2 die x-Koordinaten der dem x-Wert des Maximum $f^{max}$ am nächsten gelegenen Stellen im Kurvenverlauf sind, an denen f(x) einhalbmal $f^{max}$ ist, und H1 links und H2 rechts von diesem x-Wert liegen.

Schließlich wird erfindungsgemäß die Signalintensität $I_B$ auch durch folgende Formel beschrieben:

$$I_B = \int\limits_{D1}^{D2} f(x)dx$$

wobei

$$D1 = x - (x - x\_links)/2$$

und

$$D2 = x + (x\_rechts - x)/2,$$

wobei

x die x-Koordinate der Position A der zu bestimmenden Signalintensität
x_links die x-Koordinate des Maximums des Peaks links davon und
x_rechts die x-Koordinate des Maximums des Peaks rechts davon ist.

Letztlich kann erfindungsgemäß die Signalintensität $I_B$ eines Signals an einer Position A wiederum als Fläche unter der Signalkurve nach folgender Formel berechnet werden:

$$I_B = \int\limits_{X1}^{X2} f(x)dx$$

wobei als Grenzwerte X1 und X2 die x-Koordinaten einer beliebigen Kombination der bereits beschriebenen Werte, W1, W2, H1, H2, M1, M2, D1 oder D2 benutzt werden können, jedoch X1 links und X2 rechts von dem x-Wert liegen müssen, der die Position A bestimmt.

Erfindungsgemäß ist auch ein Verfahren in dem man die Sequenzanalyse nach der Kettenabbruchmethode durchführt.

Erfindungsgemäß ist weiterhin besonders bevorzugt, dass man in Schritt b) eine Sequenzanalyse für mindestens zwei unterschiedliche Basen durchführt und wobei sich ein weiterer Schritt anschließt, in welchem man eine Normierung der durchschnittlichen Signalintensität mindestens einer Basenart (C, T, A oder G) gegenüber der durchschnittlichen Signalintensität, die für eine oder mehrere der übrigen Basenarten erhalten wird, vornimmt, wobei man den Durchschnitt über eine Vielzahl von Positionen dieser Basenart innerhalb eines beliebig definierten Bereichs des Amplifikats ermittelt. Unter einer Vielzahl von Positionen wird im Zusammenhang mit dieser Erfindung mindestens zwei Positionen verstanden. Es können aber auch mehr als zwei, bis hin zu einer Zahl bis zu 100 Positionen und mehr. Maßgebend ist dabei, dass die Durchschnittsbildung zu mathematisch vernünftigen und/oder statistisch verlässlichen Werten führt.

Dabei ist weiterhin bevorzugt, dass man für die Bestimmung der genannten Umwandlungsrate in Schritt c) zuvor normierte Signalintensitäten verwendet.

Besonders bevorzugt ist dabei ferner, dass man für die Berechnung des tatsächlichen Methylierungsgrades des zu untersuchenden Cytosins sowohl die normierten Signalintensitäten als auch die unter Berücksichtigung der normierten Signalintensitäten erhaltene Umwandlungsrate verwendet.

Erfindungsgemäß ist es dabei ganz besonders bevorzugt, dass man die Signalintensität der Cytosin-Signale gegenüber der Signalintensität der Thymin-Signale normiert oder respektive beim Heranziehen der Sequenzinformationen des revers komplementären Stranges die Signalintensität der Guanin-Signale gegenüber der Signalintensität der Adenin-Signale normiert.

Erfindungsgemäß berechnet sich der Faktor zur Normierung $f_N$ aus

$$f_N = <I_C^{CG}> / (<I_T^T> - <I_T^{CG}>)$$

oder aus dessen Kehrwert

$$f_N^{-1} = (<I_T^T> - <I_T^{CG}>) / <I_C^{CG}>$$

oder respektive bei Heranziehung der Sequenzinformation des revers-komplementären Stranges

$$f_N = <I_G^{CG}> / (<I_A^A> - <I_A^{CG}>)$$

oder aus dessen Kehrwert

$$f_N^{-1} = (<I_A^A> - <I_A^{CG}>) / <I_G^{CG}>$$

wobei $<I_T^T>$ die durchschnittliche Thymin-Signalintensität ($I_T$) und $<I_A^A>$ die durchschnittliche Adenin-Signalintensität ($I_A$) an Positionen, die auch vor Bisulfit-Behandlung Thymin, respektive Adenin waren; wobei $<I_T^{CG}>$ und $<I_C^{CG}>$ die durchschnittlichen Thymin- und Cytosin-Signalintensitäten an Cytosinen in CpG - Positionen; und wobei $<I_A^{CG}>$ und $<I_G^{CG}>$ respektive die durchschnittlichen Adenin- und Guanin-Signalintensitäten an Guaninen in CpG - Positionen wiedergeben; und wobei der Durchschnitt jeweils über alle berücksichtigten Positionen innerhalb eines beliebig definierten Sequenz-Bereiches gebildet wird.

Erfindungsgemäß berechnet sich der Faktor zur Normierung $f_N$ aber auch aus

$$f_N = <I_C^C> / (<I_T^T> - <I_T^C>)$$

oder aus dessen Kehrwert

$$f_N^{-1} = (<I_T^T> - <I_T^C>) / <I_C^C>$$

oder respektive bei Heranziehung der Sequenzinformation des revers-komplementären Stranges

$$f_N = <I_G{}^G> \; / \; (<I_A{}^A> - <I_A{}^G>)$$

oder aus dessen Kehrwert

$$f_N{}^{-1} = (<I_A{}^A> - <I_A{}^G>) \; / \; <I_G{}^G>$$

wobei $<I_T{}^T>$ die durchschnittliche Thymin-Signalintensität ($I_T$) und $<I_A{}^A>$ die durchschnittliche Adenin-Signalintensität ($I_A$) an Positionen, die auch vor Bisulfit-Behandlung Thymin, respektive Adenin waren, wiedergibt;
wobei $<I_T{}^C>$ und $<I_C{}^C>$ die durchschnittlichen Thymin- und Cytosin-Signalintensitäten an Cytosinen und $<I_A{}^G>$ und $<I_G{}^G>$ die durchschnittliche Adenin- und Guanin-Signalintensitäten an Guaninen wiedergeben, die nicht in CpG - Positionen stehen, und wobei der Durchschnitt jeweils über alle berücksichtigten Positionen innerhalb eines beliebig definierten Sequenz-Bereiches gebildet wird.

Weiterhin berechnet sich der Faktor zur Normierung $f_N$ erfindungsgemäß auch aus

$$f_N = <I_T{}^{A,G}> \; / \; <I_C{}^{A,G}>$$

oder aus dessen Kehrwert

$$f_N{}^{-1} = <I_C{}^{A,G}> \; / \; <I_T{}^{A,G}>$$

oder respektive bei Heranziehung der Sequenzinformation des revers-komplementären Stranges

$$f_N = <I_A{}^{T,C}> \; / \; <I_G{}^{T,C}>$$

oder aus dessen Kehrwert

$$f_N{}^{-1} = <I_G{}^{T,C}> \; / \; <I_A{}^{T,C}>$$

wobei $<I_T{}^{A,G}>$ die durchschnittliche Thymin-Signalintensität ($I_T$) und $<I_C{}^{A,G}>$ die durch-schnittliche Cytosin-Signalintensität ($I_C$) an Adenin- oder Guanin-Positionen wiedergibt;
und wobei respektive $<I_A{}^{T,C}>$ die durchschnittliche Adenin-Signalintensität ($I_A$) und $<I_G{}^{T,C}>$ die durchschnittliche Guanin-Signalintensität ($I_G$) an Thymin- oder Cytosin-Positionen wiedergibt; und wobei der Durchschnitt jeweils über alle berücksichtigten Positionen innerhalb eines beliebig definierten Sequenz-Bereiches gebildet wird.

Erfindungsgemäß wird die Umwandlungsrate $f_{CON}$ berechnet aus

$$f_{CON} = I_T{}^C \; / \; (f_N I_C{}^C + I_T{}^C)$$

bzw.

$$f_{CON} = <I_T{}^C \; / \; (f_N I_C{}^C + I_T{}^C)>$$

oder aus dessen Kehrwert

$$f_{CON}{}^{-1} = (f_N I_C{}^C + I_T{}^C) \; / \; I_T{}^C$$

bzw.

$$f_{CON}^{-1} = <(f_N I_C^C + I_T^C) \ / \ I_T^C>$$

oder respektive bei Heranziehung der Sequenzinformation des revers-komplementären Stranges

$$f_{CON} = I_A^G \ / \ (f_N I_G^G + I_A^G)$$

bzw.

$$f_{CON} = <I_A^G \ / \ (f_N I_G^G + I_A^G)>$$

oder aus dessen Kehrwert

$$f_{CON}^{-1} = (f_N I_G^G + I_A^G) \ / \ I_A^G$$

bzw.

$$f_{CON}^{-1} = <(f_N I_G^G + I_A^G) \ / \ I_A^G>$$

wobei, je nachdem welche Normierung angewandt wird, $f_N$ einer der oben genannten Normierungsfaktoren ist; wobei $I_T^C$ die Thymin-Signalintensität und $I_C^C$ die Cytosin-Signalintensität eines nicht in einer CpG Position befindlichen Cytosins und respektive $I_A^G$ die Adenin-Signalintensität und $I_G^G$ die Guanin-Signalintensität eines nicht in einer CpG Position befindlichen Guanins, nach Bisulfit-Behandlung wiedergibt; und wobei entweder der Durchschnitt über die entsprechenden Quotienten aller berücksichtigten Positionen innerhalb eines beliebig definierten Sequenz-Bereiches gebildet oder nur eine Cytosin-, respektive Guanin-Position berücksichtigt wird.

Erfindungsgemäß errechnet sich die Umwandlungsrate $f_{CON}$ aber auch aus

$$f_{CON} = <I_T^C> \ / \ <I_T^T>$$

oder aus dessen Kehrwert

$$f_{CON}^{-1} = <I_T^T> \ / \ <I_T^C>$$

oder respektive bei Heranziehung der Sequenzinformation des revers-komplementären Stranges

$$f_{CON} = <I_A^G> \ / \ <I_A^A>$$

oder aus dessen Kehrwert

$$f_{CON}^{-1} = <I_A^A> \ / \ <I_A^G>$$

wobei $<I_T^C>$ die durchschnittliche Thymin-Signalintensität eines nicht in einer CpG Position befindlichen Cytosins und $<I_A^G>$ die durchschnittliche Adenin-Signalintensität eines nicht in einer CpG Position befindlichen Guanins nach Bisulfit-Behandlung wiedergibt und $<I_T^T>$ die durch-schnittliche Thymin-Signalintensität an einer Thyminposition und $<I_A^A>$ die durchschnittliche Adenin-Signalintensität an einer Adeninposition wiedergibt und wobei der Durchschnitt über alle berücksichtigten Positionen innerhalb eines beliebig definierten Sequenz-Bereiches gebildet wird.

Erfindungsgemäß errechnet sich der Methylierungsgrad $f_{MET}$ nach folgender Formel

$$f_{MET} = 1 - I_T^{CG} / (f_{CON}(f_N I_C^{CG} + I_T^{CG}))$$

oder respektive bei Heranziehung der Sequenzinformation des revers-komplementären Stranges

$$f_{MET} = 1 - I_A^{CG} / (f_{CON}(f_N I_G^{CG} + I_A^{CG}))$$

wobei $I_T^{CG}$ und $I_C^{CG}$ die Thymin- und Cytosin-Signalintensität des untersuchten Cytosins in CpG-Position und $I_A^{CG}$ und $I_G^{CG}$ respektive die Adenin- und Guanin-Signalintensität des untersuchten Guanins in CpG-Position wiedergeben; und wobei ein Methylierungsgrad von 1 einer 100%igen Methylierung entspricht.

Erfindungsgemäß errechnet sich der Methylierungsgrad $f_{MET}$ auch nach folgender Formel

$$f_{MET} = 1 - I_T^{CG} / f_{CON}<I_T^{T}>$$

oder respektive bei Heranziehung der Sequenzinformation des revers-komplementären Stranges

$$f_{MET} = 1 - I_A^{CG} / f_{CON}<I_A^{A}>$$

wobei $I_T^{CG}$ die Thymin-Signalintensität eines Cytosin in CpG-Position und $<I_T^{T}>$ die durchschnittliche Thymin-Signalintensität an Thymin-Positionen wiedergibt; und wobei $I_A^{CG}$ respektive die Adenin-Signalintensität eines Guanin in CpG-Position und $<I_A^{A}>$ die durchschnittliche Adenin-Signalintensität an Adenin-Positionen wiedergibt; und wobei ein Methylierungsgrad von 1 einer 100% igen Methylierung entspricht.

Weiterhin ist erfindungsgemäß ein Verfahren besonders bevorzugt, in dem die Aneinanderausrichtung einer Abfolge von Basen nur einer Basensorte, an eine nur zum Teil bekannte mindestens drei verschiedene Nukleotide beinhaltende Nukleotidsequenz nach Ermittlung der Abstände zweier beliebiger einzelner Basen unter Zuhilfenahme statistischer und signal-theoretischer Verfahren, mit Hilfe von probabilistischen Verfahren erfolgt.

Unter den allgemeinen erfinderischen Gedanken fallen somit zwei Verfahrenvarianten, welche erfindungsgemäß wie folgt ausgestaltet sind.

Zum einen ein Verfahren zur Bestimmung des Methylierungsgrades eines zu untersuchenden Cytosins im Genom wenigstens einer Zelle oder wenigstens eines Organismus, in dem man die folgenden Schritte ausführt:

a) Behandlung einer DNA-Probe mit Bisulfit und anschließende Amplifizierung eines ausgewählten Fragments dieser behandelten DNA;

b) Durchführung einer Sequenzanalyse des Amplifikats, in welcher die unterschiedlichen Basen der DNA entweder in räumlich getrennten Spuren analysiert oder aber durch unterschiedliche Farbmarkierung unterscheidbar innerhalb einer Spur analysiert werden, und in welcher das Ergebnis in Form eines Elektropherogramms dargestellt wird;

c) Normierung der durchschnittlichen Signalintensität mindestens einer Basenart (C, T, A oder G) gegenüber der durchschnittlichen Signalintensität, die für eine oder mehrere der übrigen Basenarten erhalten wird, wobei der Durchschnitt über eine Vielzahl von Positionen dieser Basenart innerhalb eines beliebig definierten Bereichs des Amplifikats ermittelt wird;

d) Bestimmung einer Umwandlungsrate von Cytosin in Uracil in Folge von Bisulfit-Behandlung, dadurch gekennzeichnet, dass mindestens eine in Schritt c) normierte Signalintensität an Positionen, die ihr Hybridisierungsverhalten durch Bisulfit-Behandlung ändern, zu mindestens einer anderen Signalintensität ins Verhältnis gesetzt wird, wobei hierzu sowohl die sense-Sequenz als auch deren reverskomplementäre Sequenz herangezogen werden kann;

e) Berechnung des tatsächlichen Methylierungsgrades des zu untersuchenden Cytosins unter Berücksichtigung der normierten Signalintensitäten und der in Schritt d) bestimmten Umwandlungsrate.

Und zum anderen ein Verfahren zur Bestimmung des Methylierungsgrades eines zu untersuchenden Cytosins im Genom wenigstens einer Zelle oder eines Organismus, in dem man die folgenden Schritte ausführt:

a) Behandlung einer DNA-Probe mit Bisulfit und anschließende Amplifizierung eines ausgewählten Fragments dieser behandelten DNA;

b) Durchführung einer Sequenzanalyse des Amplifikats, in welcher nur die Thymin- oder im revers-komplementären Strang nur die Adenin-Sequenz der DNA ermittelt und das Ergebnis in Form eines Elektropherogramms dargestellt wird;

c) Bestimmung einer Umwandlungsrate von Cytosin in Uracil in Folge von Bisulfit-Behandlung, dadurch gekennzeichnet, dass die Signalintensität von Basen, die ihr Hybridisierungsverhalten durch Bisulfit-Behandlung ändern, zu der Signalintensität der Basen ins Verhältnis gesetzt wird, die sich nicht durch Bisulfit-Behandlung verändern, wobei hierzu sowohl die sense-Sequenz als auch deren reverskomplementäre Sequenz herangezogen werden kann;

d) Berechnung des tatsächlichen Methylierungsgrades des zu untersuchenden Cytosins unter Berücksichtigung der in Schritt c) bestimmten Umwandlungsrate.

Die Erfindung wird nachfolgend näher beschrieben. Die Erfindung beschreibt ein Verfahren zur quantitativen Sequenzierung von mit Bisulfit behandelten Nukleinsäuren, genauer gesagt, ein Verfahren zur Bestimmung des Methylierungsgrades eines oder mehrerer Cytosine in der Basenfolge 5'-CG-3', also in einer sogenannten CpG-Position, wenn die genomische Sequenz der untersuchten DNA Region bekannt ist. Im Besonderen beschreibt die Erfindung ein Verfahren zur Bestimmung des Anteils an Nukleinsäuremolekülen, die in CpG Position methyliert sind, aus einem Gemisch von Nukleinsäuren, die methylierte und nicht methylierte Cytosine aufweisen, basierend auf einer Sequenzanalyse des mit Bisulfit behandelten Gemischs von Nukleinsäuren. Die Erfindung ermöglicht die Validierung von Sequenzinformation bei Sequenzierung von mit Bisulfit behandelter DNA. Insbesondere wird beschrieben, wie die mit üblichen Sequenzierungsmethoden erhaltenen Daten zunächst hinsichtlich ihrer Signalintensität normiert werden, wie aus diesen Daten die Umwandlungsrate von Cytosin zu Uracil in Folge von Bisulfit-Behandlung bestimmt wird und wie mit Hilfe dieser Umwandlungsrate eine wesentlich verbesserte Abschätzung des tatsächlich vorliegenden Methylierungsgrades gelingt.

In der vorliegenden Erfindung wird im Einzelnen dargestellt, wie man sowohl Signalintensitäten von Elektropherogrammen gegeneinander normiert als auch anhand der im Elektropherogramm enthaltenen Information die Umwandlungsrate der Bisulfitreaktion berechnet. Es wird beschrieben, wie sich mit Hilfe dieser Faktoren eine wesentlich genauere Aussage über den Anteil an methylierten Cytosinen in einem Gemisch von methylierten und nicht-methylierten Cytosinen getroffen werden kann. Damit erweist sich die Erfindung als überaus nützlich zur Analyse von Methylierungsgraden von DNA-Proben und verbessert damit zum Beispiel auch die Diagnose von methylierungsspezifischen Erkrankungen erheblich.

Die Erfindung erstreckt sich auf die Analyse von DNA Gemischen sowohl in einzelnen Zellen als auch von DNA Gemischen aus einer Vielzahl von Zellen, welche entweder aus einem Organismus oder aus verschiedenen Organismen stammen können. Die Methode zur Bestimmung des Methylierungsgrades wenigstens eines zu untersuchenden Cytosins im Genom wenigstens einer solchen Zelle, die in dieser Anmeldung beschrieben ist, setzt voraus, dass die genomische Nukleotidsequenz der nicht mit Bisulfit behandelten DNA bekannt ist. Im ersten Schritt der Methode wird die zu untersuchende genomische DNA-Probe mit Bisulfit behandelt, wobei nicht methylierte Cytosin-Basen so verändert werden, dass sie in der anschließenden PCR ein Hybridisierungsverhalten wie Thymin-Basen aufweisen, während die 5-Methylcytosinbasen unverändert bleiben. Die durch PCR erhaltenen, doppelsträngigen DNA Amplifikate enthalten anstelle eines unmethylierten Cytosins im sense-Strang ein Thymin und entsprechend auf dem revers komplementären Strang ein Adenin. Folglich lässt sich durch Ermittlung der Thymin-Signalintensitäten an ursprünglichen Cytosin-Positionen in CpG-Stellung bestimmen, wie groß der Anteil der unmethylierten Cytosine an dieser CpG-Position in dem vorliegenden Gemisch ist.

Gegenstand der Erfindung ist eine erste erfindungsgemäße Verfahrenvariante, bei der man die Amplifikate zunächst sequenziert und die markierten Reaktionsprodukte nachfolgend ihrer Größe nach entweder in räumlich getrennten Spuren oder aber durch unterschiedliche Farbmarkierung unterscheidbar innerhalb einer Spur analysiert. Es können zum Beispiel vier verschiedene Fluoreszenz-markierte ddNTPs verwendet werden; es ist aber auch möglich die Analyse auf die Ermittlung von weniger als vier Basenabfolgen zu beschränken. Die Sequenzanalyse endet dem Stand der Technik entsprechend mit dem Erhalt der Elektropherogramme, welche üblicherweise nur zur qualitativen Bestimmung der Basenabfolge herangezogen werden. In der vorliegenden Erfindung ist beschrieben, wie aus diesem Elektropherogramm und aus dem Vergleich dieser Daten mit der Original-Sequenz, also der Sequenz der nicht mit Bisulfit behandelteten DNA, auch quantitative Aussagen bezüglich des Methylierungsgrades eines Cytosins gewonnen werden können.

Zunächst erfolgt hierfür eine Normierung der Elektropherogramm-Daten. Die durchschnittliche Signalintensität mindestens einer Basenart (C, T, A oder G), wird zu der durchschnittlichen Signalintensität einer oder mehrerer der übrigen Basenarten ins Verhältnis gesetzt. In einer besonders bevorzugten Variante der Methode, wird die Signalintensität von Cytosin gegenüber der von Thymin, ins Verhältnis gesetzt. Der Durchschnitt berechnet sich hierbei jeweils über die Signalintensitätswerte der Basen, die man in einem beliebig definierten Bereich des Amplifikats vorfindet. Dieser kann das gesamte Amplifikat, oder aber auch nur einen kleinen Ausschnitt dessen umfassen.

Aus den normierten Signalintensitäten wird im nächsten Schritt eine Umwandlungsrate von Cytosin in Uracil in Folge von Bisulfit-Behandlung ( $f_{CON}$ ) berechnet. Sie beschreibt das Verhältnis der unmethylierten Cytosinbasen, deren Hybridisierungsverhalten durch Bisulfitbehandlung in das Hybridisierungsverhalten von Thymin verändert wurde, zu allen unmethylierten Cytosinbasen, unabhängig davon ob deren Hybridisierungsverhalten verändert wurde oder nicht, innerhalb eines definierten Sequenz-Bereichs. Der zu berücksichtigende Bereich kann die Länge des gesamten Amplifikats oder aber nur einen Teil dessen umfassen.

Unter Berücksichtigung der Normierung und mit Hilfe dieser Umwandlungsrate $f_{CON}$ wird im letzten Schritt die wirkliche, oder wenigstens ein der Realität näherliegender Methylierungsgrad $f_{MET}$ bestimmt.

Gegenstand der Erfindung ist eine zweite, verwandte Verfahrensvariante zur Bestimmung dieses Methylierungsgrads, für den Fall, dass neben der bekannten genomischen Nukleotidsequenz der nicht mit Bisulfit behandelten DNA, dessen Kenntnis Voraussetzung zur Bestimmung der Methylierungsgrades ist, nur die Thymin-Elektropherogramme der mit Bisulfit behandelten DNA zur Analyse herangezogen werden sollen oder können. Es ist Gegenstand der Erfindung, wie ohne die Kenntnis um die Cytosin-, Guanin- und Adenin-Basenabfolgen allein aus dem Thymin-Elektropherogramm zunächst eine Umwandlungsrate $f_{CON}$ bestimmt werden kann und wie sich mit deren Hilfe der Methylierungsgrad berechnen lässt. Für diesen Fall ist die Berechnung der Umwandlungsrate von umso größerer Bedeutung, da eine Normierung der Daten weder möglich noch notwendig ist.

Bevorzugt sind Varianten beider Methoden, dadurch gekennzeichnet, dass die Cytosin-Signalintensitäten gegenüber den Thymin-Signalintensitäten normiert werden.

Die Berechnung der Normierungsfaktoren, zur Normierung von Signalintensitäten, sowie die Berechnung der Umwandlungsrate basieren auf möglichst genauer Kenntnis der Signalintensitäten selbst.

In einer bevorzugten Variante der ersten Methode werden Normierungsfaktoren wie folgt berechnet:

Da das Elektropherogramm einen Kurvenverlauf darstellt, der die Anzahl der detektierten Signale pro Zeiteinheit darstellt, welche wiederum (als inhärentes Merkmal der Sequenzierungsmethode) den räumlichen Abstand zwischen zwei Basen widerspiegelt, lässt sich die Signalintensität und damit die Anzahl der Moleküle die jenes Signal tragen, durch die Fläche unter dem Peak, also unter dem lokalen Maximum dieses Kurvenverlaufs berechnen. Durch Integrierung dieses Kurvenverlaufs lässt sich die berücksichtigte Fläche am besten beschreiben. Diese Flächenmaße werden durch die Integrationsgrenzen X1 und X2 festgelegt. Die Integrale werden jeweils von einem Wert X1 links des lokalen Maximums liegend, bis zu einem Wert X2, rechts des lokalen Maximums liegend, gebildet. Zur Berechnung der Signalintensität sind hierin für die Integrierung der Flächen unter den lokalen Maxima folgende möglichen Grenzwerte besonders bevorzugt.

Das Verfahren ist dementsprechend besonders bevorzugt wenn die Maßzahlen für die Signalintensität entweder durch

- Integrierung der Fläche unter dem Signalverlauf von dem dem lokalen Maximum links am nächsten gelegenen Wendepunkt zu dem ihm rechts am nächsten liegenden Wendepunkt,
- Integrierung der Fläche unter dem Signalverlauf von dem dem lokalen Maximum links am nächsten gelegenen lokalen Minimum zu dem ihm rechts am nächsten liegenden Minimum,
- Integrierung der Fläche unter dem Signalverlauf von dem dem lokalen Maximum links am nächsten gelegenen Halbe-Höhe-Punkt, also der x-Koordinate des Wertes, der die halbe Höhe (1/2 f(x) ) des lokalen Maximums beträgt, zu dem rechts des lokalen Maximums am nächsten liegenden Halbe-Höhe Punktes,
- Integrierung der Fläche unter dem Signalverlauf von einem Wert D1 zu einem Wert D2, wobei D1 und D2 die x-Koordinaten beschreiben, die jeweils genau mittig zwischen zwei lokalen Maxima liegen, wobei sich D1 links des zu bestimmenden Maximums und D2 rechts des zu bestimmenden Maximums befindet,
- oder durch Integrierung der Fläche unter dem Signalverlauf durch Einbeziehung einer beliebigen Kombination der beschriebenen Grenzwerte,

erfolgt.

Bevorzugt ist eine Variante beider Verfahren, die dadurch gekennzeichnet ist, dass die verwendete Bisulfit-SequenzInformation mit Hilfe der Kettenabbruch-Methode bestimmt wurde.

Besonders bevorzugt ist ein Verfahren, in dem sich der Normierungsfaktor aus dem Verhältnis der durchschnittlichen Cytosin-Signalintensität an Cytosinen in CpG-Position und der Differenz aus der durchschnittlichen Thymin-Signalintensität an Thyminen und der durchschnittlichen Thymin-Signalintensität an Cytosinen in CpG-Position berechnen lässt:

$$f_N = \langle I_C^{CG} \rangle \ / \ (\langle I_T^{T} \rangle - \langle I_T^{CG} \rangle)$$

oder aus dessen Kehrwert

$$f_N^{-1} = (<I_T^T> - <I_T^{CG}>) / <I_C^{CG}>$$

Es ist offensichtlich, und damit in der bevorzugten Variante inbegriffen, dass sich Thymin- und Cytosin-Signalintensitäten hierin durch Adenin- und Guanin-Signalintensitäten ersetzen lassen, falls zur Durchführung der Methode die Sequenzdaten des komplementär reversen Stranges herangezogen werden:

$$f_N = <I_G^{CG}> / (<I_A^A> - <I_A^{CG}>)$$

oder aus dessen Kehrwert

$$f_N^{-1} = (<I_A^A> - <I_A^{CG}>) / <I_G^{CG}>$$

Ganz besonders bevorzugt ist eine Variante des ersten Verfahrens, das dadurch gekennzeichnet ist, dass sich der Normierungsfaktor aus dem Verhältnis der durchschnittlichen Cytosin-Signalintensität an Cytosinen außerhalb von CpG-Positionen und der Differenz der durchschnittlichen Thymin-Signalintensität an Thyminen und der durchschnittlichen Thymin-Signalintensität an Cytosinen außerhalb von CpG-Position berechnen lässt:

$$f_N = <I_C^C> / (<I_T^T> - <I_T^C>)$$

oder aus dessen Kehrwert

$$f_N^{-1} = (<I_T^T> - <I_T^C>) / <I_C^C>$$

Es ist auch hier offensichtlich, und damit in der bevorzugten Variante inbegriffen, dass sich Thymin- und Cytosin-Signalintensitäten hierin durch Adenin- und Guanin-Signalintensitäten ersetzen lassen, falls zur Durchführung der Methode die Sequenzdaten des komplementär reversen Stranges herangezogen werden:

$$f_N = <I_G^G> / (<I_A^A> - <I_A^G>)$$

oder aus dessen Kehrwert

$$f_N^{-1} = (<I_A^A> - <I_A^G>) / <I_G^G>$$

Besonders bevorzugt ist außerdem eine Variante des Verfahrens, die dadurch gekennzeichnet ist, dass sich der Normierungsfaktor aus dem Verhältnis der durchschnittlichen Thymin- und Cytosin-Signalintensitäten an Adenin- und Guanin-Positionen berechnen lässt:

$$f_N = <I_T^{A,G}> / <I_C^{A,G}>$$

oder aus dessen Kehrwert

$$f_N^{-1} = <I_C^{A,G}> / <I_T^{A,G}>$$

Umgekehrt gilt bei Heranziehung der Bisulfit-Sequenzdaten des komplementär reversen Stranges, dass die Berechnung dieses Normierungsfaktors aus dem Verhältnis der durchschnittlichen Adenin- und Guanin-Signalintensitäten an Thymin- und Cytosin-Positionen bevorzugt ist:

$$f_N = <I_A^{T,C}> / <I_G^{T,C}>$$

oder aus dessen Kehrwert

$$f_N^{-1} = <I_G^{T,C}> / <I_A^{T,C}>$$

Für alle genannten Berechnungen der Durchschnittswerte gilt, dass diese immer über einen festgelegten SequenzBereich gebildet werden, der sowohl die Länge des gesamten Amplifikats umfassen als auch aus nur einer Mindestzahl an Basen bestehen kann.

Die Methode beinhaltet in einem weiteren Schritt die Bestimmung einer Umwandlungsrate $f_{CON}$.

Insbesondere bevorzugt ist eine Variante der ersten Methode, dadurch gekennzeichnet, dass eine Umwandlungsrate $f_{CON}$ aus dem Verhältnis von in Thymin umgewandelten Cytosinen und der Summe aller, umgewandelter und nicht umgewandelter, Cytosine innerhalb eines definierten Sequenz-bereichs berechnet wird. Dazu wird das Verhältnis aus den Thymin-Signalintensitäten an Positionen, die vor der Bisulfit-Behandlung durch Cytosin besetzt waren und keine CpG-Positionen sind und der Summe dieser Thymin- und der normierten Cytosin-Signalintensitäten an besagten Positionen berechnet:

$$f_{CON} = I_T^C / (f_N I_C^C + I_T^C)$$

bzw.

$$f_{CON} = <I_T^C / (f_N I_C^C + I_T^C)>$$

oder aus dessen Kehrwert

$$f_{CON}^{-1} = (f_N I_C^C + I_T^C) / I_T^C$$

bzw.

$$f_{CON}^{-1} = <(f_N I_C^C + I_T^C) / I_T^C>$$

oder respektive bei Heranziehung der Sequenzinformation des revers-komplementären Stranges:

$$f_{CON} = I_A^G / (f_N I_G^G + I_A^G)$$

bzw.

$$f_{CON} = \langle I_A^G / (f_N I_G^G + I_A^G) \rangle$$

oder aus dessen Kehrwert

$$f_{CON}^{-1} = (f_N I_G^G + I_A^G) / I_A^G$$

bzw.

$$f_{CON}^{-1} = \langle (f_N I_G^G + I_A^G) / I_A^G \rangle$$

Außerdem ist eine Variante beider Methoden bevorzugt, die dadurch gekennzeichnet ist, dass sich diese Umwandlungsrate auch einzig unter Zuhilfenahme der Thymin-Signalintensitäten berechnen lässt. Dazu wird das Verhältnis der Thymin-Signalintensität an einer Position, die vor Bisulfitbehandlung durch Cytosin besetzt war, sich jedoch nicht in einer CpG-Position befindet, und der Thymin-Signalintensität an einer Thymin-Position gebildet.

$$f_{CON} = \langle I_T^C \rangle / \langle I_T^T \rangle$$

oder aus dessen Kehrwert

$$f_{CON}^{-1} = \langle I_T^T \rangle / \langle I_T^C \rangle$$

oder respektive bei Heranziehung der Sequenzinformation des revers-komplementären Stranges

$$f_{CON} = \langle I_A^G \rangle / \langle I_A^A \rangle$$

oder aus dessen Kehrwert

$$f_{CON}^{-1} = \langle I_A^A \rangle / \langle I_A^G \rangle$$

Es gelten für alle genannten bevorzugten Varianten der Verfahren zur Berechnung der Umwandlungsrate, dass hierfür entweder Durchschnittswerte über mehrere solcher Positionen in einem definierten Sequenzbereich hinweg oder aber Einzelwerte herangezogen, um damit entweder die Umwandlungsrate für einen Sequenzbereich oder die Umwandlungsrate eines einzelnen Cytosins zu bestimmen.

Ebenfalls bevorzugt ist eine Variante der ersten Methode, die dadurch gekennzeichnet ist, dass mit Hilfe des ermittelten Normierungsfaktors und der Umwandlungsrate für jedes Cytosin innerhalb des untersuchten Sequenzbereiches, welches sich in einer CpG-Position befindet, ein Methylierungsgrad $f_{MET}$ berechnet wird, die sowohl die mitunter eingeschränkte Effizienz der Bisulfitreaktion, als auch die zum Teil stark verfälschten Signalintensitäten berücksichtigt. Dies gelingt durch das Anwenden folgender Formel:

$$f_{MET} = 1 - I_T^{CG} / (f_{CON}(f_N I_C^{CG} + I_T^{CG}))$$

Hierin beziehen sich alle Signalintensitäten I auf das Cytosin in CpG-Position: $I^{CG}$. $I_T^{CG}$ beschreibt die Signalintensität des Thymins an diesem Cytosin, also den Anteil der umgewandelten Cytosine. Dieser Wert wird durch Einbeziehen der Konversionsrate $f_{CON}$ dem realen Wert angenähert. $I_C^{CG}$ beschreibt die Cytosin-Signalintensität an diesem Cytosin in CpG-Position. $I_C^{CG}$ beschreibt sowohl die methylierten Cytosine als auch die nicht methylierten, aber nicht umgewandelten Cytosine in CpG-Position und wird durch Einbeziehen eines Normierungsfaktors $f_N$ dem realen Wert angenähert. Letztlich beschreibt $f_{MET}$ genau die Rate an tatsächlich methylierten Cytosinen, wobei $f_{MET}$ Werte zwischen 0 und 1 annehmen kann.

Ist das Cytosin in der CpG-Position vollständig methyliert (Methylierung 100%), entspricht dies einer $f_{MET}$ von 1. Dieser Fall liegt dann vor, wenn kein Thymin-Signal erhalten wird (also $I_T^{CG} = 0$). Dann gilt:

$$f_{MET} = 1 - 0 \; / \; (f_{CON}(f_N I_C^{CG} + 0)) = 1$$

Ist das Cytosin in der CpG-Position nicht methyliert (Methylierung 0%), entspricht dies einer $f_{MET}$ von 0. Bei einer 100%ig erfolgten Umwandlung, also einer Umwandlungsrate $f_{CON} = 1$, wird kein Cytosin-Signal erhalten und es gilt : $f_N I_C^{CG} = 0$. Damit wird:

$$f_{MET} = 1 - I_T^{CG} \; / \; (f_{CON} I_T^{CG}) = 1 - 1/f_{CON} = 0$$

hat jedoch keine 100%ige Umsetzung stattgefunden, werden immer auch Cytosin-Signale erhalten, selbst wenn die Cytosine nicht methyliert sind.

Bei einer nur zu 50% methylierten Cytosinposition und zu 100% erfolgter Umwandlung sind die Anteile an normiertem Cytosin-Signal und Thymin-Signal gleich groß. Es gilt :

$$f_{MET} = 1 - I_T^{CG} \; / \; (f_{CON}(f_N I_C^{CG} + I_T^{CG}))$$

$$f_{MET} = 1 - I_T^{CG} \; / \; (f_{CON}(2 * I_T^{CG}))$$

$$f_{MET} = 1 - 1 \; / \; (f_{CON} * 2)$$

$$f_{MET} = 1 - 1 \; / \; 2$$

Immer gilt, dass bei Heranziehung der Sequenzinformation des revers-komplementären Stranges die selben Formeln zur Anwendung kommen, aber alle Thymine durch Adenine und alle Cytosine durch Guanine ersetzt werden müssen, also zum Beispiel :

$$f_{MET} = 1 - I_A^{CG} \; / \; (f_{CON}(f_N I_G^{CG} + I_A^{CG}))$$

Außerdem bevorzugt ist eine Variante der zweiten Methode, dadurch gekennzeichnet dass, beim Zugrundeliegen nur einer Basenabfolge, nämlich der Thyminsequenz, der mit Bisulfit behandelten DNA ein Methylierungsgrad $f_{MET}$ nach folgender Formel berechnet wird:

$$f_{MET} = 1 - I_T^{CG} \; / \; f_{CON} \langle I_T^{T} \rangle$$

Hierzu wird auf die durchschnittliche Thymin-Signalintensität an Thyminen $< I_T^T >$ zurückgegriffen. Respektive gilt bei Heranziehung der Sequenzinformation des revers-komplementären Stranges diese Formel

$$f_{MET} = 1 - I_A^{CG} / f_{CON} <I_A^A>$$

wobei auf die durchschnittliche Adenin-Signalintensität an Adeninen $< I_A^A >$ zurückgegriffen wird.

In beiden Fällen gilt, dass $f_{MET}$ Werte zwischen 0 und 1 annehmen kann und ein Methylierungsgrad von 1 einer 100%igen Methylierung entspricht.

Bei der Variante der Methode, die beim Vorliegen nur einer Basenabfolge, nämlich der Thyminsequenz oder respektive, bei Heranziehen des revers komplementären Stranges, der Adeninsequenz, angewandt wird, ergibt sich die Schwierigkeit, die Signalintensitäten eindeutig zuordnen zu können. Eine Sequenz, die über einen längeren Bereich keine Thymine und gegebenenfalls nur minimale Signale, sogenannte Fehlersignale, aufweist, ist seiner bekannten genomischen Nukleotidsequenz nur schwer zuzuordnen. Noch schwieriger ist dies, wenn man berücksichtigt, dass die vorliegende Einbasensequenz einer mit Bisulfit behandelten Nukleotidsequenz zugeordnet werden muss, die nur bedingt bekannt ist.

Daher wird, was diesen weiteren Gegenstand der Erfindung betrifft, eine Variante der Methode besonders bevorzugt, in der zunächst mit statistischen und signaltheoretischen Methoden der Abstand zwischen zwei Peaks, also zwischen zwei potentiellen Basen-Positionen ermittelt wird und daraufhin mit Hilfe probabilistischer Verfahren die Zuordnung zu den bekannten, aus der genomischen Sequenz abgeleiteten Positionen vorgenommen wird.

Die folgenden Beispiele erläutern die Erfindung.

Beispiel 1:

Untersuchung des Methylierungsgrades des Gens p16 mittels Sequenzanalyse.

Das folgende Beispiel bezieht sich auf ein Fragment des Gens p16, in dem bestimmte CpG-Positionen hinsichtlich ihres Methylierungsgrades untersucht werden. In diesem Beispiel wurde die DNA kommerziell erhalten und vom Anbieter zuvor aus menschlichem Blut isoliert.

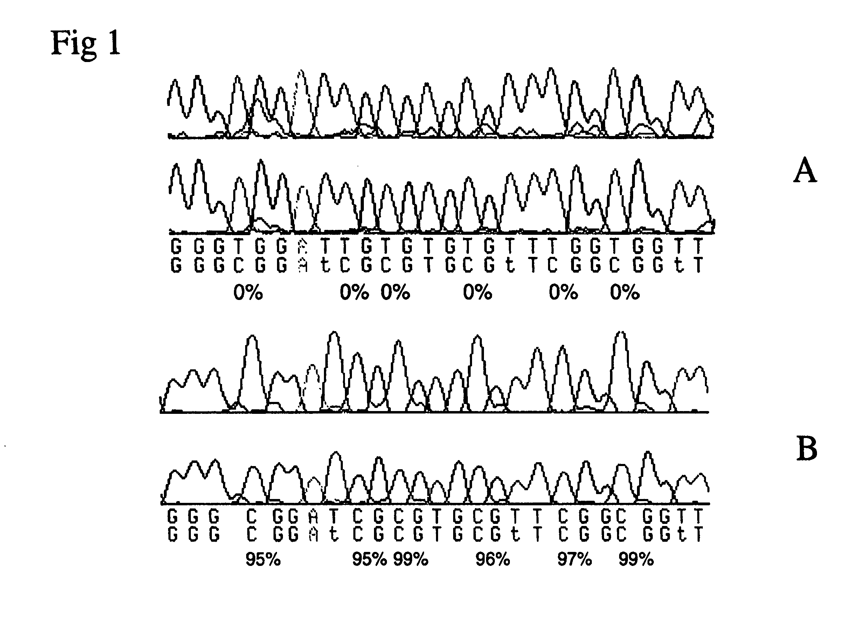

Im ersten Schritt wird eine genomische Sequenz unter Verwendung von Bisulfit (Hydrogensulfit, Disulfit) derart behandelt, dass alle nicht an der 5-Position der Base methylierten Cytosine so verändert werden, dass eine hinsichtlich dem Basenpaarungsverhalten unterschiedliche Base entsteht, während die in 5-Position methylierten Cytosine unverändert bleiben. Wird für die Reaktion Bisulfit verwendet, so findet an den nicht methylierten Cytosinbasen eine Addition statt. Zudem müssen ein denaturierendes Reagenz oder Lösungsmittel sowie ein Radikalfänger zugegen sein. Eine anschließende alkalische Hydrolyse führt dann zur Umwandlung von nicht methylierten Cytosinen in Uracil. Diese umgewandelte DNA dient dazu, methylierte Cytosine von nicht methylierten Cytosinen zu unterscheiden und diese damit nachzuweisen. Im zweiten Verfahrensschritt verdünnt man die behandelte DNA-Probe mit Wasser oder einer wässrigen Lösung. Bevorzugt wird anschließend eine Desulfonierung der DNA durchgeführt. In einem weiteren Schritt amplifiziert man die DNA-Probe in einer Polymerasekettenreaktion, bevorzugt mit einer hitzebeständigen DNA-Polymerase. Im vorliegenden Fall werden Cytosine des Gens p16 untersucht. Dazu wird mit den spezifischen Primer-Oligonukleotiden P1 (siehe Seq-ID1) und P2 (siehe Seq-ID 2) ein definiertes Fragment von 256 bp Länge amplifiziert. Das vorliegende PCR-Produkt ist ein DNA Gemisch aus Amplifikaten, die entweder Cytosine oder Thymine an den zu untersuchenden CpG-Positionen aufweisen. Dieses Gemisch aus Amplifikaten dient als Templat für eine nachfolgende zyklische Sequenzierreaktion. Für die Sequenzierreaktion werden die gleichen Primeroligonukleotide wie für die Polymerasekettenreaktion verwendet. Das Ergebnis der Sequenzierung ist in Abbildung 1 in Form von Elektopherogrammen wiedergegeben.

In Figur 1 sind im oberen Teil (Elektropherogramm-Paar A) zwei Elektropherogramme dargestellt, die einen Teilbereich der Sequenz des Gens p16, in dem Methylierungszustand, der beim kommerziellen Erwerb des DNA Gemisches gegeben war, abbilden. Im unteren Teil der Abbildung (Elektropherogramm-Paar B) sind zwei Elektropherogramme dargestellt, die den selben Sequenzbereich darstellen.

Der Unterschied zu dem oberen Abbildungsteil besteht darin, dass die DNA, die dieser Sequenzierung zugrunde liegt, in einem vorgeschalteten Schritt auf-methyliert wurde. Die Methylierungsgrade der Cytosine erreichen hiernach beinahe 100%.

Die jeweils oberen Elektopherogramme der Elektropherogramm-Paare A und B beschreiben die Sequenzen, wie sie von der software des Sequenzierautomaten generiert werden. Sie zeigen also ein herkömmlich erhaltenes Elektropherogramm. Die jeweils unteren Elektropherogramme der Elektropherogramm-Paare A und B beschreiben die Sequenzen, wie sie nach der Anwendung der hierin beschriebenen Erfindung dargestellt werden. Das Auftauchen von Fremdsignalen, also das Untergrund-Rauschen ist deutlich reduziert.

Unterhalb der Elektopherogramme werden zwei Sequenzen als Buchstabenfolge angezeigt, welche die jeweiligen Basen indizieren. Die jeweils obere Basenabfolge bezieht sich auf das untere, also optimierte Elektropherogramm, die jeweils untere Basenabfolge hingegen zeigt die theoretisch erwartete Sequenz, wie sie bei bekannter genomischer Sequenz nach Bisulfitbehandlung erwartet wird und dient zum Vergleich der durch den Sequenzierautomaten generierten Daten.

A steht für Adenin, C steht für Cytosin, G steht für Guanin, T steht für Thymin und t steht an Basenpositionen, die erst nach Bisulfitbehandlung das Hybridisierungsverhalten eines Thymins zeigen, zuvor, in der genomischen Sequenz jedoch Cytosine waren. ( In der farbigen Darstellung sind die Basen in vier verschiedenen Farben dargestellt: Thymin = T = Rot, Cytosin = C = Blau, Adenin = A = Grün, Guanin = G = Schwarz.)

Unterhalb der Basenabfolge sind Zahlen abgebildet. Sie geben den jeweiligen prozentualen Methylierungsgrad eines Cytosins in CpG-Position an, wie er sich nach Anwendung der hierin beschriebenen Methode berechnet.

Beispiel 2:

Darstellung von Elektropherogrammen nach Bisulfit-Sequenzierung mit und ohne Verwendung des erfindungsgemäßen Verfahrens:

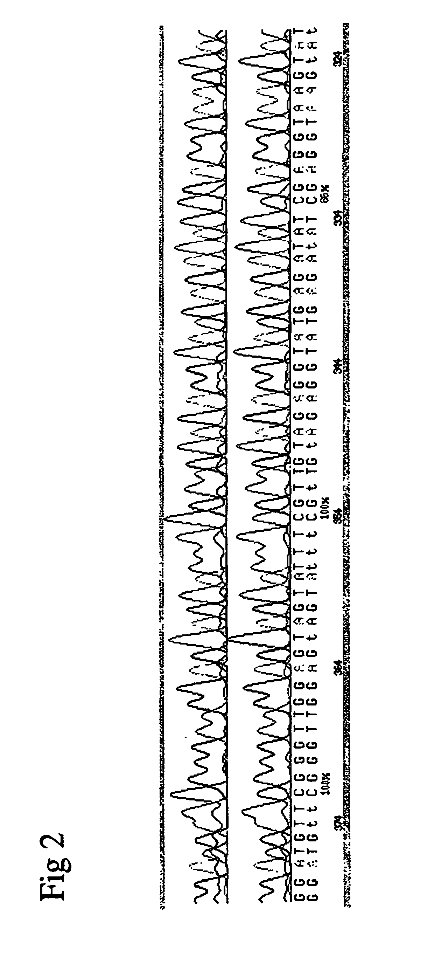

In Figur 2 sind zwei komplexe Elektropherogramme übereinander abgebildet. Die Zahlen am unteren Rand der Abbildung beschreiben die Basenposition innerhalb des sequenzierten Amplifikats und dienen der Orientierung. In Abbildung 2 erstrecken sich beide Elektropherogramme von Base 322 bis Base 379. Das obere Elektropherogramm beschreibt die Abfolge der vier verschiedenen Basen (in vier verschiedenen Farben : Thymin = T = Rot, Cytosin = C = Blau, Adenin = A = Grün, Guanin = G = Schwarz) ohne Normierung oder Berücksichtigung der Umwandlungsrate, also ohne die vorliegende Erfindung anzuwenden. Das darunter liegende Elektropherogramm beschreibt die Daten, wie sie sich nach Anwendung einer Variante der hierin beschriebenen Erfindung darstellen. Es ist deutlich zu erkennen, daß die Cytosin-Signale im oberen Elektropherogramm alle wesentlich größer sind als im unteren, nachdem die Cytosin-Signalintensität normiert wurde. Ebenso wird auch das "Rauschen" geringer, zum Beispiel gut erkennbar in dem Bereich 373 bis 379 bp. Die Prozentangaben unterhalb von Cytosinen in CpG-Position geben den Methylierunggrad des Cytosins nach Berechnung anhand der hier beschriebenen Methode an.

## Patentansprüche

1.  Verfahren zur Bestimmung des Methylierungsgrades eines zu untersuchenden Cytosins, innerhalb eines CpG-Dinukleotides, welches sich innerhalb eines Sequenz-bereichs des Genoms befindet, dessen Sequenz bekannt ist, im Genom wenigstens einer Zelle oder eines Organismus, **dadurch gekennzeichnet, dass** man die folgenden Schritte ausführt:

    a) Behandlung einer DNA-Probe mit Bisulfit und anschließende Amplifizierung eines ausgewählten Fragments dieser behandelten DNA;
    b) Durchführung einer Sequenzanalyse des Amplifikats, in welcher man das Ergebnis in Form eines Elektropherogramms darstellt;
    c) Bestimmung einer Umwandlungsrate von unmethyliertem Cytosin in Uracil in Folge von Bisulfit-Behandlung, **dadurch gekennzeichnet, dass** die Signalintensität von Basen, die ihr Hybridisierungsverhalten durch Bisulfit-Behandlung ändern, zu der Signalintensität der Basen ins Verhältnis gesetzt wird, die sich nicht durch Bisulfit-Behandlung verändern, wobei man hierzu die bereits bekannte sense-Sequenz und/oder deren revers-komplementäre Sequenz heranzieht;
    d) Berechnung des tatsächlichen Methylierungsgrades des zu untersuchenden Cytosins durch Bestimmung des Anteils an methylierten DNA-Molekulen in einen Gemisch aus methylierten und nicht-methylierten DNA-Molekülen unter Berücksichtigung der in Schritt c) bestimmten Umwandlungsrate.

2.  Verfahren nach Anspruch 1, **dadurch gekennzeichnet, dass** man in der Sequenzanalyse des Amplifikats nur die Thymin- oder im revers-komplementären Strang nur die Adenin-Sequenz der DNA ermittelt und das Ergebnis in Form eines Elektropherogramms darstellt.

3.  Verfahren nach den Ansprüchen 1 oder 2, in der die Signalintensität $I_B$ einer Base B, wobei B entweder C, A, G oder T sein kann und damit die Basenart festlegt, deren Intensität gemeint ist, als Teil der Fläche unter dem Peak im Elektropherogramm durch folgende Formel beschrieben wird, wobei f(x) den Kurvenverlauf des Intensitätssignals über einer Einheit x beschreibt, welche die Zeit wiedergibt, die nach chromatographischer Trennung den Abstand zwischen zwei Basen beschreibt:

$$I_B = \int\limits_{W1}^{W2} f(x)dx$$

wobei W1 und W2 die x-Koordinaten der dem x-Wert des Maximum $f^{max}$ am nächsten gelegenen Wendepunkte sind und W1 links und W2 rechts von diesem x-Wert liegen.

4. Verfahren nach Anspruch 1 oder 2, in der die Signalintensität $I_B$ durch folgende Formel beschrieben wird:

$$I_B = \int\limits_{M1}^{M2} f(x)dx$$

wobei M1 und M2 die x-Koordinaten der dem x-Wert des Maximum $f^{max}$ am nächsten gelegenen Minima sind und M1 links und M2 rechts von diesem x-Wert liegen.

5. Verfahren nach Anspruch 1 oder 2, in der die Signalintensität $I_B$ durch folgende Formel beschrieben wird:

$$I_B = \int\limits_{H1}^{H2} f(x)dx$$

wobei H1 und H2 die x-Koordinaten der dem x-Wert des Maximum $f^{max}$ am nächsten gelegenen Stellen im Kurvenverlauf sind, an denen f(x) einhalbmal $f^{max}$ ist, und H1 links und H2 rechts von diesem x-Wert liegen.

6. Verfahren nach Anspruch 1 oder 2, in der die Signalintensität $I_B$ durch folgende Formel beschrieben wird:

$$I_B = \int\limits_{D1}^{D2} f(x)dx$$

wobei

```
D1 = x - (x - x_links)/2
```

und

```
D2 = x + (x_rechts - x)/2,
```

wobei

    x die x-Koordinate der Position A der zu bestimmenden Signalintensität
    x_links die x-Koordinate des Maximums des Peaks links davon und
    x_rechts die x-Koordinate des Maximums des Peaks rechts davon ist.

**7.** Verfahren nach den Ansprüchen 1 oder 2, in der die Signalintensität $I_B$ eines Signals an einer Position A wiederum als Fläche unter der Signalkurve nach folgender Formel berechnet wird:

$$I_B = \int_{X1}^{X2} f(x)\,dx$$

wobei als Grenzwerte X1 und X2 die x-Koordinaten einer beliebigen Kombination der bereits beschriebenen Werte, W1, W2, H1, H2, M1, M2, D1 oder D2 benutzt werden können, jedoch X1 links und X2 rechts von dem x-Wert liegen müssen, der die Position A bestimmt.

**8.** Verfahren nach den Ansprüchen 1 oder 2, in der man die Sequenzanalyse nach der Kettenabbruchmethode durchführt.

**9.** Verfahren nach Anspruch 1, **dadurch gekennzeichnet, dass** man in Schritt b) eine Sequenzanalyse für mindestens zwei unterschiedliche Basen durchführt und wobei sich ein weiterer Schritt anschließt, in welchem man eine Normierung der durchschnittlichen Signalintensität mindestens einer Basenart (C, T, A oder G) gegenüber der durchschnittlichen Signalintensität, die für eine oder mehrere der übrigen Basenarten erhalten wird, vornimmt, wobei man den Durchschnitt über mindestens zwei Positionen dieser Basenart innerhalb eines beliebig definierten Bereichs des Amplifikats ermittelt.

**10.** Verfahren nach Anspruch 9, **dadurch gekennzeichnet, dass** man für die Bestimmung der genannten Umwandlungsrate in Schritt c) zuvor normierte Signalintensitäten verwendet.

**11.** Verfahren nach Anspruch 9, **dadurch gekennzeichnet, dass** man für die Berechnung des tatsächlichen Methylierungsgrades des zu untersuchenden Cytosins sowohl die normierten Signalintensitäten als auch die unter Berücksichtigung der normierten Signalintensitäten erhaltene Umwandlungsrate verwendet.

**12.** Verfahren nach Anspruch 9, **dadurch gekennzeichnet, dass** man die Signalintensität der Cytosin-Signale gegenüber der Signalintensität der Thymin-Signale normiert oder respektive beim Heranziehen der Sequenzinformationen des revers komplementären Stranges die Signalintensität der Guanin-Signale gegenüber der Signalintensität der Adenin-Signale normiert.

**13.** Verfahren nach Anspruch 9, worin sich der Faktor zur Normierung $f_N$ berechnet aus

$$f_N = \langle I_C^{CG} \rangle \,/\, (\langle I_T^{T} \rangle - \langle I_T^{CG} \rangle)$$

oder aus dessen Kehrwert

$$f_N^{-1} = (\langle I_T^{T} \rangle - \langle I_T^{CG} \rangle )\,/\,\langle I_C^{CG} \rangle$$

oder respektive bei Heranziehung der Sequenzinformation des revers-komplementären Stranges

$$f_N = <I_G^{CG}> \; / \; (<I_A^{A}> \; - \; <I_A^{CG}>)$$

oder aus dessen Kehrwert

$$f_N^{-1} = (<I_A^{A}> \; - \; <I_A^{CG}>) \; / \; <I_G^{CG}>$$

wobei $<I_T^{T}>$ die durchschnittliche Thymin-Signalintensität $(I_T)$ und $<I_A^{A}>$ die durchschnittliche Adenin-Signalintensität $(I_A)$ an Positionen, die auch vor Bisulfit-Behandlung Thymin, respektive Adenin waren;
wobei $<I_T^{CG}>$ und $<I_C^{CG}>$ die durchschnittlichen Thymin- und Cytosin-Signalintensitäten an Cytosinen in CpG - Positionen; und wobei $<I_A^{CG}>$ und $<I_G^{CG}>$ respektive die durchschnittlichen Adenin- und Guanin-Signalintensitäten an Guaninen in CpG - Positionen wiedergeben; und
wobei der Durchschnitt jeweils über alle berücksichtigten Positionen innerhalb eines beliebig definierten Sequenz-Bereiches gebildet wird.

**14.** Verfahren nach Anspruch 9, worin sich der Faktor zur Normierung $f_N$ berechnet aus

$$f_N = <I_C^{C}> \; / \; (<I_T^{T}> \; - \; <I_T^{C}>)$$

oder aus dessen Kehrwert

$$f_N^{-1} = (<I_T^{T}> \; - \; <I_T^{C}>) \; / \; <I_C^{C}>$$

oder respektive bei Heranziehung der Sequenzinformation des revers-komplementären Stranges

$$f_N = <I_G^{G}> \; / \; (<I_A^{A}> \; - \; <I_A^{G}>)$$

oder aus dessen Kehrwert

$$f_N^{-1} = (<I_A^{A}> \; - \; <I_A^{G}>) \; / \; <I_G^{G}>$$

wobei $<I_T^{T}>$ die durchschnittliche Thymin-Signalintensität $(I_T)$ und $<I_A^{A}>$ die durchschnittliche Adenin-Signalintensität $(I_A)$ an Positionen, die auch vor Bisulfit-Behandlung Thymin, respektive Adenin waren, wiedergibt;
wobei $<I_T^{C}>$ und $<I_C^{C}>$ die durchschnittlichen Thymin- und Cytosin-Signalintensitäten an Cytosinen und $<I_A^{G}>$ und $<I_G^{G}>$ die durchschnittliche Adenin- und Guanin-Signalintensitäten an Guaninen wiedergeben, die nicht in CpG - Positionen stehen, und wobei der Durchschnitt jeweils über alle berücksichtigten Positionen innerhalb eines beliebig definierten Sequenz-Bereiches gebildet wird.

**15.** Verfahren nach Anspruch 9, worin sich der Faktor zur Normierung $f_N$ berechnet aus

$$f_N = <I_T^{A,G}> / <I_C^{A,G}>$$

oder aus dessen Kehrwert

$$f_N^{-1} = <I_C^{A,G}> / <I_T^{A,G}>$$

oder respektive bei Heranziehung der Sequenzinformation des revers-komplementären Stranges

$$f_N = <I_A^{T,C}> / <I_G^{T,C}>$$

oder aus dessen Kehrwert

$$f_N^{-1} = <I_G^{T,C}> / <I_A^{T,C}>$$

wobei $<I_T^{A,G}>$ die durchschnittliche Thymin-Signalintensität ($I_T$) und $<I_C^{A,G}>$ die durchschnittliche Cytosin-Signalintensität ($I_C$) an Adenin- oder Guanin-Positionen wiedergibt; und wobei respektive $<I_A^{T,C}>$ die durchschnittliche Adenin-Signalintensität ($I_A$) und $<I_G^{T,C}>$ die durchschnittliche Guanin-Signalintensität ($I_G$) an Thymin- oder Cytosin-Positionen wiedergibt; und wobei der Durchschnitt jeweils über alle berücksichtigten Positionen innerhalb eines beliebig definierten Sequenz-Bereiches gebildet wird.

**16.** Verfahren nach Anspruch 9, worin sich die Umwandlungsrate $f_{CON}$ berechnet aus

$$f_{CON} = I_T^C / (f_N I_C^C + I_T^C)$$

bzw.

$$f_{CON} = <I_T^C / (f_N I_C^C + I_T^C)>$$

oder aus dessen Kehrwert

$$f_{CON}^{-1} = (f_N I_C^C + I_T^C) / I_T^C$$

bzw.

$$f_{CON}^{-1} = <(f_N I_C^C + I_T^C) / I_T^C>$$

oder respektive bei Heranziehung der Sequenzinformation des revers-komplementären Stranges

$$f_{CON} = I_A^G / (f_N I_G^G + I_A^G)$$

bzw.

$$f_{CON} = <I_A^G / (f_N I_G^G + I_A^G)>$$

oder aus dessen Kehrwert

$$f_{CON}^{-1} = (f_N I_G^G + I_A^G) / I_A^G$$

bzw.

$$f_{CON}^{-1} = <(f_N I_G^G + I_A^G) / I_A^G>$$

wobei, je nachdem welche Normierung angewandt wird, $f_N$ der Normierungsfaktor nach Anspruch 13, 14 oder 15 ist; wobei $I_T^C$ die Thymin-Signalintensität und $I_C^C$ die Cytosin-Signalintensität eines nicht in einer CpG Position befindlichen Cytosins und respektive $I_A^G$ die Adenin-Signalintensität und $I_G^G$ die Guanin-Signalintensität eines nicht in einer CpG Position befindlichen Guanins, nach Bisulfit-Behandlung wiedergibt; und wobei entweder der Durchschnitt über die entsprechenden Quotienten aller berücksichtigten Positionen innerhalb eines beliebig definierten Sequenz-Bereiches gebildet oder nur eine Cytosin-, respektive Guanin-Position berücksichtigt wird.

**17.** Verfahren nach Anspruch 1, worin sich die Umwandlungsrate $f_{CON}$ berechnet aus

$$f_{CON} = <I_T^C> / <I_T^T>$$

oder aus dessen Kehrwert

$$f_{CON}^{-1} = <I_T^T> / <I_T^C>$$

oder respektive bei Heranziehung der Sequenzinformation des revers-komplementären Stranges

$$f_{CON} = <I_A^G> / <I_A^A>$$

oder aus dessen Kehrwert

$$f_{CON}^{-1} = \langle I_A^A \rangle \ / \ \langle I_A^G \rangle$$

wobei $\langle I_T^C \rangle$ die durchschnittliche Thymin-Signalintensität eines nicht in einer CpG Position befindlichen Cytosins und $\langle I_A^G \rangle$ die durchschnittliche Adenin-Signalintensität eines nicht in einer CpG Position befindlichen Guanins nach Bisulfit-Behandlung wiedergibt und $\langle I_T^T \rangle$ die durchschnittliche Thymin-Signalintensität an einer Thyminposition und $\langle I_A^A \rangle$ die durchschnittliche Adenin-Signalintensität an einer Adeninposition wiedergibt und
wobei der Durchschnitt über alle berücksichtigten Positionen innerhalb eines beliebig definierten Sequenz-Bereiches gebildet wird.

18. Verfahren nach Anspruch 9, gemäß einem der Ansprüche 13 bis 15 und einem der Ansprüche 16 oder 17, in dem sich der Methylierungsgrad $f_{MET}$ nach folgender Formel berechnet

$$f_{MET} = 1 \ - \ I_T^{CG} \ / \ (f_{CON}(f_N I_C^{CG} + I_T^{CG}))$$

oder respektive bei Heranziehung der Sequenzinformation des revers-komplementären Stranges

$$f_{MET} = 1 \ - \ I_A^{CG} \ / \ (f_{CON}(f_N I_G^{CG} + I_A^{CG}))$$

wobei $I_T^{CG}$ und $I_C^{CG}$ die Thymin- und Cytosin-Signalintensität des untersuchten Cytosins in CpG-Position und $I_A^{CG}$ und $I_G^{CG}$ respektive die Adenin- und Guanin-Signalintensität des untersuchten Guanins in CpG-Position wiedergeben,
wobei ein Methylierungsgrad von 1 einer 100%igen Methylierung entspricht und
wobei zur Berechnung eines Methylierungsgrades $f_{MET}$ die hierzu verwendeten Komponenten der Umwandlungsrate $f_{CON}$ und des Normierungsfaktors $f_N$ sich auf den jeweils selben Strang beziehen wie der ermittelte Methylierungsgrad.

19. Verfahren nach Anspruch 1, gemäß einem der Ansprüche 13 bis 15 und einem der Ansprüche 16 oder 17, in dem sich der Methylierungsgrad $f_{MET}$ nach folgender Formel berechnet

$$f_{MET} = 1 \ - \ I_T^{CG} \ / \ f_{CON} \langle I_T^T \rangle$$

oder respektive bei Heranziehung der Sequenzinformation des revers-komplementären Stranges

$$f_{MET} = 1 \ - \ I_A^{CG} \ / \ f_{CON} \langle I_A^A \rangle$$

wobei $I_T^{CG}$ die Thymin-Signalintensität eines Cytosin in CpG-Position und $\langle I_T^T \rangle$ die durchschnittliche Thymin-Signalintensität an Thymin-Positionen wiedergibt; und wobei $I_A^{CG}$ respektive die Adenin-Signalintensität eines Guanin in CpG- Position und $\langle I_A^A \rangle$ die durchschnittliche Adenin-Signalintensität an Adenin-Positionen wiedergibt, wobei ein Methylierungsgrad von 1 einer 100%igen Methylierung entspricht und
wobei zur Berechnung eines Methylierungsgrades $f_{MET}$ die hierzu verwendeten Komponenten der Umwandlungsrate $f_{CON}$ und des Normierungsfaktors $f_N$ sich auf den jeweils selben Strang beziehen wie der ermittelte Methylierungsgrad.

20. Verfahren nach Anspruch 2, in dem die Aneinanderausrichtung einer Abfolge von Basen nur einer Basensorte, an eine, dieser Sequenz in nicht umgewandelter DNA entsprechende, nur zum Teil bekannte und mindestens drei verschiedene Nukleotide beinhaltende Nukleotidsequenz nach Ermittlung der Abstände zweier beliebiger einzelner Basen unter Zuhilfenahme statistischer und signal-theoretischer Verfahren, mit Hilfe von probabilistischen Verfahren erfolgt.

**Claims**

1. A method for determining the degree of methylation of a cytosine to be investigated, within a CpG dinucleotide which is located within a sequence region of the genome, the sequence of which is known, within the genome of at least one cell or one organism, **characterized in that** the following steps are conducted:

   a) Treatment of a DNA sample with bisulfite and subsequent amplification of a selected fragment of this treated DNA;
   b) Conducting a sequence analysis of the amplificate, in which the result is represented in the form of an electropherogram;
   c) Determination of a conversion rate of unmethylated cytosine to uracil as a consequence of bisulfite treatment, **characterized in that** a ratio of the signal intensity of bases which modify their hybridization behavior due to bisulfite treatment to the signal intensity of bases which do not modify their hybridization behavior due to bisulfite treatment, wherein one utilizes the sense sequence already known and/or its inversely complementary sequence;
   d) Calculation of the actual degree of methylation of the cytosine to be investigated by determinating the fraction of methylated DNA molecules in a mixture of methylated DNA molecules in a mixture of methylated and unmethylated DNA molecules, taking into consideration the conversion rate determined in step c).

2. The method according to claim 1, **characterized in that** one determines only the thymine sequence, or in the inversely complementary strand only the adenine sequence, of the DNA in the sequence analysis of the amplificate and presents the result in the form of an electropherogram.

3. The method according to claims 1 or 2, in which the signal intensity $I_B$ of a base B, wherein B can be either C, A, G or T, and thus establishes the type of base whose intensity is determined, is described as a part of the area under the peak in the electropherogram by the following formula, wherein f(x) describes the curve of the intensity signal over a unit x, which represents the time, which describes the distance between two bases after chromatographic separation.

$$I_B = \int_{W1}^{W2} f(x)dx$$

wherein W1 and W2 are the x-coordinates of the points of inflection lying closest to the x value of the maximum f^max and W1 lies to the left and W2 to the right of this x value.

4. The method according to claim 1 or 2, in which the signal intensity $I_B$ is described by the following formula:

$$I_B = \int_{M1}^{M2} f(x)dx$$

wherein M1 and M2 are the x-coordinates of the minima lying closest to the x value of the maximum f^max and M1 lies to the left and M2 to the right of this x value.

5. The method according to claim 1 or 2, in which the signal intensity $I_B$ is described by the following formula:

$$I_B = \int_{H1}^{H2} f(x)\,dx$$

wherein H1 and H2 are the x-coordinates of the positions in the curve at which f(x) is one-half $f^{max}$, lying closest to the x value of the maximum $f^{max}$, and H1 lies to the left and H2 to the right of this x value.

6. The method according to claim 1 or 2, in which the signal intensity $I_B$ is described by the following formula:

$$I_B = \int_{D1}^{D2} f(x)\,dx$$

wherein

```
D1 = x - (x - x_left) /2
```

and

```
D2 = x + (x_right - x)/2,
```

wherein

x is the x-coordinate of the position A of the signal intensity to be determined
x_left is the x-coordinate of the maximum of the peak to the left thereof and
x_right is the x-coordinate of the maximum of the peak to the right thereof.

7. The method according to claims 1 or 2, in which the signal intensity $I_B$ of a signal at a position A can be calculated as the area under the signal curve according to the following formula:

$$I_B = \int_{X1}^{X2} f(x)\,dx$$

wherein the x-coordinates of a random combination of the already described values, W1, W2, H1, H2, M1, M2, D1 or D2 can be used as limiting values X1 and X2, but X1 must lie to the left and X2 to the right of the x-value that determines position A.

8. The method according to claims 1 or 2, in which the sequence analysis is conducted according to the chain termination method.

9. The method according to claim 1, further **characterized in that** a sequence analysis of at least two different bases is conducted in step b), and wherein another step follows,
in which a standardizing of the average signal intensity of at least one base type (C, T, A or G) is conducted against the average signal intensity which is obtained for one or more of the remaining base types,
wherein the average of at least two positions of this base type is determined within a randomly defined region of the amplificate.

10. The method according to claim 9, **characterized in that** previously standardized signal intensities are used for the determination of said conversion rates in step c).

**11.** The method according to claim 9, **characterized in that** both the standardized signal intensities as well as the conversion rates obtained taking into consideration the standardized signal intensities are used for the calculation of the actual degree of methylation of the cytosine to be investigated.

**12.** The method according to claim 9, further **characterized in that** the signal intensity of the cytosine signals is standardized against the signal intensity of the thymine signals or, when utilizing the sequence information of the inversely complementary strand, the signal intensity of the guanine signals is standardized against the signal intensity of the adenine signals.

**13.** The method according to claim 9, wherein the factor for standardizing $f_N$ is calculated from

$$f_N = <I_C^{CG}> / (<I_T^T> - <I_T^{CG}>)$$

or from its reciprocal value

$$f_N^{-1} = (<I_T^T> - <I_T^{CG}>) / <I_C^{CG}>$$

or, respectively, when utilizing the sequence information of the inversely complementary strand,

$$f_N = <I_G^{CG}> / (<I_A^A> - <I_A^{CG}>)$$

or from its reciprocal value

$$f_N^{-1} = (<I_A A> - <I_A^{CG}>) / <I_G^{CG}>$$

wherein $<I_T^T>$ represents the average thymine signal intensity ($I_T$) and $<I_A^A>$ represents the average adenine signal intensity ($I_A$) at positions, which were also thymine or adenine prior to bisulfite treatment;
wherein $<I_T^{CG}>$ and $<I_C^{CG}>$ are the average thymine and cytosine signal intensities at cytosines in CpG positions;
and wherein $<I_A^{CG}>$ and $<I_G^{CG}>$, respectively, represent the average adenine and guanine signal intensities at guanines in CpG positions; and
wherein the average of all of the considered positions is taken each time within a randomly defined sequence region.

**14.** The method according to claim 9, wherein the factor for standardizing $f_N$ is calculated from

$$f_N = <I_C^C> / (<I_T^T> - <I_T^C>)$$

or from its reciprocal value

$$f_N^{-1} = (<I_T^T> - <I_T^C>) / <I_C^C>$$

or, respectively, when utilizing the sequence information of the inversely complementary strand,

$$f_N = <I_G{}^G> / (<I_A{}^A> - <I_A{}^G>)$$

or from its reciprocal value

$$f_N{}^{-1} = (<I_A{}^A> - <I_A{}^G>) / <I_G{}^G>$$

wherein $<I_T{}^T>$ represents the average thymine signal intensity $(I_T)$ and $<I_A{}^A>$ represents the average adenine signal intensity $(I_A)$ at positions, which were also thymine or adenine prior to bisulfite treatment; wherein $<I_T{}^C>$ and $<I_C{}^C>$ represent the average thymine and cytosine signal intensities at cytosines and $<I_A{}^G>$ and $<I_G{}^G>$ represent the average adenine and guanine signal intensities at guanines, which do not stand in CpG positions, and wherein the average of all of the considered positions is taken each time within a randomly defined sequence region.

**15.** The method according to claim 9, wherein the factor for standardizing $f_N$ is calculated from

$$f_N = <I_T{}^{A,G}> / <I_C{}^{A,G}>$$

or from its reciprocal value

$$f_N{}^{-1} = <I_C{}^{A,G}> / <I_T{}^{A,G}>$$

or, respectively, when drawing on the sequence information of the inversely complementary strand,

$$f_N = <I_A{}^{T,C}> <I_G{}^{T,C}>$$

or from its reciprocal value

$$f_N{}^{-1} = <I_G{}^{T,C}> / <I_A{}^{T,C}>$$

wherein $<I_T{}^{A,G}>$ represents the average thymine signal intensity $(I_T)$ and $<I_C{}^{A,G}>$ the average cytosine signal intensity $(I_C)$ at adenine or guanine positions; and wherein $<I_A{}^{T,C}>$, respectively, represents the average adenine signal intensity $(I_A)$, and $<I_G{}^{T,C}>$ represents the average guanine signal intensity $(I_G)$ at thymine or cytosine positions; and wherein the average of all of the considered positions is taken each time within a randomly defined sequence region.

**16.** The method according to claim 9, wherein the conversion rate $f_{CON}$ is calculated from

$$f_{CON} = I_T{}^C / (f_N I_C{}^C + I_T{}^C)$$

or

$$f_{CON} = \langle I_T^C / f_N I_C^C + I_T^C) \rangle$$

or from its reciprocal value

$$f_{CON}^{-1} = (f_N I_C^C + I_T^C) / I_T^C$$

or

$$f_{CON}^{-1} = \langle (f_N I_C^C + I_T^C) / I_T^C \rangle$$

or, respectively, when drawing on the sequence information of the inversely complementary strand,

$$f_{CON} = I_A^G / ( f_N I_G^G + I_A^G)$$

or

$$f_{CON} = \langle I_A^G / (f_N I_G^G + I_A^G) \rangle$$

or from its reciprocal value

$$f_{CON}^{-1} = (f_N I_G^G + I_A^G) / I_A^G$$

or

$$f_{CON}^{-1} = \langle (f_N I_G^G + I_A^G) / I_A^G \rangle$$

wherein, each time depending on which standardization is applied, $f_N$ is the standardization factor according to claim 13, 14 or 15; wherein $I_T^C$ represents the thymine signal intensity and $I_C^C$ represents the cytosine signal intensity of a cytosine not found in a CpG position, and respectively, $I_A^G$ represents the adenine signal intensity and $I_G^G$ represents the guanine signal intensity of a guanine not found in a CpG position, after bisulfite treatment; and wherein either the average of the corresponding quotients of all considered positions within a randomly defined sequence region is taken or only one cytosine or guanine position is considered.

**17.** The method according to claim 1, wherein the conversion rate $f_{CON}$ is calculated from

$$f_{CON} = \langle I_T^C \rangle / \langle I_T^T \rangle$$

or from its reciprocal value

$$f_{CON}^{-1} = <I_T^T> / <I_T^C>$$

or, respectively, when drawing on the sequence information of the inversely complementary strand,

$$f_{CON} = < I_A^G> / <I_A^A>$$

or from its reciprocal value

$$f_{CON}^{-1} = <I_A^A> / <I_A^G>$$

wherein $<I_T^C>$ represents the average thymine signal intensity of a cytosine not found in a CpG position and $<I_A^G>$ represents the average adenine signal intensity of a guanine not found in a CpG position, after bisulfite treatment and $<I_T^T>$ represents the average thymine signal intensity at a thymine position and $<I_A^A>$ represents the average adenine signal intensity at an adenine position, and
wherein the average of all of the considered positions is taken within a randomly defined sequence region.

18. The method according to claim 9, according one of the claims 13 to 15 and one of the claims 16 or 17, in which the methylation number $f_{MET}$ is calculated according to the following formula

$$f_{MET} = 1 - I_T^{CG} / (f_{CON} (f_N I_C^{CG} + I_T^{CG}))$$

or, respectively, when drawing on the sequence information of the inversely complementary strand,

$$f_{MET} = 1 - I_A^{CG} / (f_{CON} (f_N I_G^{CG} + I_A^{CG}))$$

wherein $I_T^{CG}$ and $I_C^{CG}$ represent the thymine and cytosine signal intensities of the investigated cytosine in the CpG position and $I_A^{CG}$ and $I_G^{CG}$, respectively, represent the adenine and guanine signal intensities of the investigated guanine in CpG position;
wherein a methylation number of 1 corresponds to a 100% methylation and
wherein for the calculation of a methylation number $f_{MET}$ the components of the conversation rate $f_{CON}$ and the factor of standardization $f_N$ utilized therefor relate to the same strand, respectively, as the methylation number determined.

19. The method according to claim 1, according to one of the claims 13 to 15 and one of the claims 16 or 17, in which the methylation number $f_{MET}$ is calculated according to the following formula

$$f_{MET} = 1 - I_T^{CG} / f_{CON}<I_T^T>$$

or, respectively, when drawing on the sequence information of the inversely complementary strand,

$$f_{MET} = 1 - I_A^{CG} / f_{CON}<I_A^A>$$

wherein $I_T^{CG}$ represents the thymine signal intensity of a cytosine in CpG position and $<I_T^T>$ represents the average thymine signal intensity at thymine positions; and wherein $I_A^{CG}$, respectively, represents the adenine signal intensity of a guanine in CpG position and $<I_A^A>$ represents the average adenine signal intensity at adenine positions, wherein a methylation number of 1 corresponds to a 100% methylation und

wherein for the calculation of a methylation number $f_{MET}$ the components of the conversation rate $f_{CON}$ and the factor of standardization $f_N$ utilized therefor relate to the same strand, respectively, as the methylation number determined.

20. The method according to claim 2, in which the alignment next to one another of a sequence of bases of only one base type at a nucleotide sequence containing at least three different nucleotides that are only partially known is produced by means of the probabilistic method after determining the distances between two random, individual bases with the help of statistical and theoretical signal methods.

**Revendications**

1. Procédé de détermination du degré de méthylation d'une cytosine à examiner à l'intérieur d'un dinucléotide CpG qui se trouve à l'intérieur d'une zone de séquence d'un génome dont la séquence est connue, dans le génome d'au moins une cellule ou d'un organisme, **caractérisé en ce que** l'on exécute les étapes suivantes :

   a) Traitement d'un échantillon d'ADN avec du bisulfure et ensuite amplification d'un fragment choisi de cet ADN traité;
   b) Réalisation d'une analyse séquentielle de l'amplificat dans lequel on représente le résultat sous la forme d'un électrophérogramme ;
   c) Détermination d'un taux de conversion de la cytosine non méthylée dans l'uracile suite au traitement au bisulfure, **caractérisé en ce que** l'intensité du signal des bases qui modifient leur comportement d'hybridation en raison du traitement au bisulfure est mis en rapport avec l'intensité du signal des baes qui ne se modifient pas par le traitement au bisulfure en utilisant à cet effet la fausse séquence déjà connue et/ou sa séquence complémentaire inversée ;
   d) Calcul du degré de méthylation réel de la cytosine à examiner en déterminant la proportion de molécules d'ADN méthylées dans un mélange de molécules d'ADN méthylées et non méthylées en tenant compte du taux de conversion déterminé dans l'étape c).

2. Procédé selon la revendication 1, **caractérisé en ce que** seule la séquence de thymine ou, dans la branche complémentaire inversée, seule la séquence d'adénine de l'ADN est déterminée dans l'analyse séquentielle de l'amplificat et le résultat est représenté sous la forme d'un électrophérogramme.

3. Procédé selon la revendication 1 ou 2, dans lequel l'intensité du signal $I_B$ d'une base B, B pouvant être C, A, G ou T et déterminant ainsi le type de base dont l'intensité est évoquée, est décrite en tant que partie de la surface sous la crête dans l'électrophérogramme par la formule suivante, f(x) décrivant le tracé de la courbe de l'intensité du signal en fonction d'une unité x indiquant le temps, laquelle décrit l'écart entre deux bases après la séparation chromatographique:

$$I_B = \int\limits_{w1}^{w2} f(x)dx$$

où w1 et w2 sont les cordonnées x du point d'inflexion le plus proche de la valeur de x du maximum $f^{max}$ et w1 se trouve à gauche et w2 à droite de cette valeur de x.

4. Procédé selon la revendication 1 ou 2, dans lequel l'intensité du signal $I_B$ est décrite par la formule suivante :

$$I_B = \int_{M1}^{M2} f(x)dx$$

où M1 et M2 sont les cordonnées x du minimum le plus proche de la valeur de x du maximum f$^{max}$ et M1 se trouve à gauche et M2 à droite de cette valeur de x.

**5.** Procédé selon la revendication 1 ou 2, dans lequel l'intensité du signal I$_B$ est décrite par la formule suivante :

$$I_B = \int_{H1}^{H2} f(x)dx$$

où H1 et H2 sont les cordonnées x du point de la courbe le plus proche de la valeur de x du maximum f$^{max}$ et auquel f(x) est égale à une fois et demi f$^{max}$ et H1 se trouve à gauche et H2 à droite de cette valeur de x.

**6.** Procédé selon la revendication 1 ou 2, dans lequel l'intensité du signal I$_B$ est décrite par la formule suivante:

$$I_B = \int_{D1}^{D2} f(x)dx$$

où

```
D1 = x - (x - x_gauche)/2
```

et

```
D2 = x + (x_droite - x)/2,
```

où

x est la cordonnée x de la position A de l'intensité du signal à déterminer
x_gauche est la coordonnée x du maximum de la crête à gauche de celle-ci et
x_droite est la coordonnée x du maximum de la crête à droite de celle-ci.

**7.** Procédé selon la revendication 1 ou 2, dans lequel l'intensité du signal I$_B$ d'un signal à une position A est de nouveau calculée sous la forme d'une surface sous la courbe du signal d'après la formule suivante :

$$I_B = \int_{X1}^{X2} f(x)dx$$

où une combinaison quelconque des valeurs déjà décrites W1, W2, H1, H2, M1, M2, D1 ou D2 peut être utilisée comme valeurs limites X1 et X2 des coordonnées x, X1 devant cependant se trouver à gauche et X2 à droite de la valeur de x qui détermine la position A.

8. Procédé selon la revendication 1 ou 2, dans lequel l'analyse séquentielle est réalisée selon la méthode de rupture de monotonie.

9. Procédé selon la revendication 1, **caractérisé en ce qu'**un analyse séquentielle d'au moins deux bases différentes est effectuée dans l'étape b) et une étape supplémentaire vient se rajouter dans laquelle on effectue une réduction de l'intensité moyenne du signal d'au moins un type de base (C, T, A ou G) par rapport à l'intensité moyenne du signal qui est obtenue pour un ou plusieurs des autres types de base, la moyenne étant déterminée sur au moins deux positions de ce type de base à l'intérieur d'une zone définie quelconque de l'amplificat.

10. Procédé selon la revendication 9, **caractérisé en ce que** les intensité de signal préalablement réduites sont utilisées pour la détermination dudit taux de conversion dans l'étape c).

11. Procédé selon la revendication 9, **caractérisé en ce que** les intensité de signal réduites ainsi que le taux de conversion obtenu en tenant compte des intensité de signal réduites sont utilisés pour le calcul du degré de méthylation réel de la cytosine à examiner.

12. Procédé selon la revendication 9, **caractérisé en ce que** l'intensité des signaux de la cytosine est réduite par rapport à l'intensité du signal de la thymine ou alors l'intensité du signal de la guanine est réduite par rapport à l'intensité du signal de l'adénine en utilisant les informations de séquence de la branche complémentaire inversée.

13. Procédé selon la revendication 9, dans lequel le facteur de réduction $f_N$, calculé à partir de

$$f_N = <I_C^{CG}> / (<I_T^T> - <I_T^{CG}>)$$

ou à partir de son inverse

$$f_N^{-1} = (<I_T^T> - <I_T^{CG}>) / <I_C^{CG}>$$

ou encore en utilisant l'information de séquence de la branche complémentaire inversée

$$f_N = <I_G^{CG}> / (<I_A^A> - <I_A^{CG}>)$$

ou à partir de son inverse

$$f_N^{-1} = (<I_A^A> - <I_A^{CG}>) / <I_G^{CG}>$$

$<I_T^T>$ étant l'intensité moyenne du signal de thymine ($I_T$) et $<I_A^A>$ l'intensité moyenne du signal d'adénine ($I_A$) aux positions qui étaient également la thymine ou l'adénine avant le traitement au bisulfure ;
$<I_T^{CG}>$ et $<I_C^{CG}>$ indiquant les intensités moyennes du signal de thymine et de cytosine aux positions de la cytosine dans CpG ; et $<I_A^{CC}>$ et $<I_G^{CG}>$ indiquant respectivement les intensités moyennes du signal d'adénine et de guanine aux positions de la guanine dans CpG ; et
la moyenne étant à chaque fois calculée sur toutes les positions considérées à l'intérieur d'une zone donnée quelconque de la séquence.

14. Procédé selon la revendication 9, dans lequel le facteur de réduction $f_N$, calculé à partir de

$$f_N = <I_C^C> / (<I_T^T> - <I_T^C>)$$

ou à partir de son inverse

$$f_N^{-1} = (<I_T^T> - <I_T^C>) / <I_C^C>$$

ou encore en utilisant l'information de séquence de la branche complémentaire inversée

$$f_N = <I_G^G> / (<I_A^A> - <I_A^G>)$$

ou à partir de son inverse

$$f_N^{-1} = (<I_A^A> - <I_A^G>) / <I_G^G>$$

$<I_T^T>$ étant l'intensité moyenne du signal de thymine ($I_T$) et $<I_A^A>$ l'intensité moyenne du signal d'adénine ($I_A$) aux positions qui étaient également la thymine ou l'adénine avant le traitement au bisulfure ;
$<I_T^C>$ et $<I_C^C>$ indiquant les intensités moyennes du signal de thymine et de cytosine au niveau des cytosines et $<I_A^G>$ et $<I_G^G>$ indiquant les intensités moyennes du signal d'adénine et de guanine au niveau des guanines, et la moyenne étant à chaque fois calculée sur toutes les positions considérées à l'intérieur d'une zone donnée quelconque de la séquence.

**15.** Procédé selon la revendication 9, dans lequel le facteur de réduction $f_N$, calculé à partir de

$$f_N = <I_T^{A,\ G}> / <I_C^{A,\ G}>$$

ou à partir de son inverse

$$f_N^{-1} = <I_C^{A,\ G}> / <I_T^{A,\ G}>$$

ou encore en utilisant l'information de séquence de la branche complémentaire inversée

$$f_N = <I_A^{T,\ C}> / <I_G^{T,\ C}>$$

ou à partir de son inverse

$$f_N^{-1} = <I_G^{T,\ C}> / <I_A^{T,\ C}>$$

$<I_T^{A,G}>$ étant l'intensité moyenne du signal de thymine ($I_T$) et $<I_C^{A,\ G}>$ l'intensité moyenne du signal de cytosine ($I_C$) aux positions de l'adénine ou de la guanine ;
$<I_A^{T,C}>$ indiquant l'intensité moyenne du signal d'adénine ($I_A$) et $<I_G^{T,C}>$ l'intensité moyenne du signal de guanine ($I_G$) aux positions de la thymine ou de la cytosine ; et
la moyenne étant à chaque fois calculée sur toutes les positions considérées à l'intérieur d'une zone donnée quelconque de la séquence.

**16.** Procédé selon la revendication 9, dans lequel le taux de conversion $f_{con}$ est calculé à partir de

$$f_{con} = I_T^C / (f_N I_C^C + I_T^C)$$

ou

EP 1 369 493 B1

$$f_{con} = \langle I_T{}^C / (f_N I_C{}^C + I_T{}^C) \rangle$$

ou à partir de leurs inverses

$$f_{con}{}^{-1} = (f_N I_C{}^C + I_T{}^C) / I_C{}^T$$

ou

$$f_{con}{}^{-1} = \langle (f_N I_C{}^C + I_T{}^C) / I_T{}^C \rangle$$

ou encore en utilisant l'information de séquence de la branche complémentaire inversée

$$f_{con} = I_A{}^G / (f_N I_G{}^G + I_A{}^G)$$

ou

$$f_{con} = \langle I_A{}^G / (f_N I_G{}^G + I_A{}^G) \rangle$$

ou à partir de leurs inverses

$$f_{con}{}^{-1} = (f_N I_G{}^G + I_A{}^G) / I_A{}^G$$

ou

$$f_{con}{}^{-1} = \langle (f_N I_G{}^G + I_A{}^G) / I_A{}^G \rangle$$

$f_N$ étant ici le facteur de réduction selon la revendication 13, 14 ou 15, suivant la réduction employée ;
$I_T{}^C$ désignant l'intensité du signal de thymine et $I_C{}^C$ l'intensité du signal de cytosine d'une cytosine qui ne se trouve pas dans une position CpG et $I_A{}^G$ l'intensité du signal d'adénine et $I_G{}^G$ l'intensité du signal de guanine d'une guanine qui ne se trouve pas dans une position CpG après le traitement au bisulfure ;
et soit la moyenne étant calculée par le biais des quotients correspondants de toutes les positions considérées à l'intérieur d'une zone donnée quelconque de la séquence ou seule est prise la position de la cytosine ou de la guanine.

17. Procédé selon la revendication 1, dans lequel le taux de conversion $f_{con}$ est calculé à partir de

$$f_{con} = \langle I_T{}^C \rangle / \langle I_T{}^T \rangle$$

ou à partir de son inverse

$$f_{con}{}^{-1} = \langle I_T{}^T \rangle / \langle I_T{}^C \rangle$$

ou encore en utilisant l'information de séquence de la branche complémentaire inversée

**34**

$$f_{con} = <I_A^G> / <I_A^A>$$

ou à partir de son inverse

$$f_{con}^{-1} = <I_A^A> / <I_A^G>$$

$<I_T^C>$ désignant l'intensité moyenne du signal de thymine d'une cytosine qui ne se trouve pas dans une position CpG et $<I_A^G>$ l'intensité moyenne du signal d'adénine d'une guanine qui ne se trouve pas dans une position CpG après le traitement au bisulfure et $<I_T^T>$ désignant l'intensité moyenne du signal de thymine à une position de thymine et $<I_A^A>$ l'intensité moyenne du signal d'adénine à une position d'adénine et
la moyenne étant calculée sur toutes les positions considérées à l'intérieur d'une zone donnée quelconque de la séquence.

**18.** Procédé selon la revendication 9, selon l'une des revendications 13 à 15 et l'une des revendications 16 ou 17, dans lequel le degré de méthylation $f_{MET}$ est calculé d'après le formule suivante

$$f_{MET} = 1 - I_T^{CG} / (f_{CON}(f_N I_C^{CG} + I_T^{CG}))$$

ou encore en utilisant l'information de séquence de la branche complémentaire inversée

$$f_{MET} = 1 - I_A^{CG} / (f_{CON}(f_N I_G^{CG} + I_A^{CG}))$$

$I_T^{CG}$ et $I_C^{CG}$ désignant l'intensité du signal de thymine et de cytosine de la cytosine examinée en position CpG et $I_A^{CG}$ et $I_G^{CG}$ désignant respectivement l'intensité du signal d'adénine et de guanine de la guanine examinée en position CpG,
un degré de méthylation de 1 correspondant à une méthylation à 100 % et
pour calculer un degré de méthylation $f_{MET}$, les composants utilisés à cet effet du taux de conversion $f_{CON}$ et du facteur de réduction $f_N$ se rapportant à chaque fois à la même branche que le degré de méthylation déterminé.

**19.** Procédé selon la revendication 1, selon l'une des revendications 13 à 15 et l'une des revendications 16 ou 17, dans lequel le degré de méthylation $f_{MET}$ est calculé d'après le formule suivante

$$f_{MET} = 1 - I_T^{CG} / f_{CON}<I_T^T>$$

ou encore en utilisant l'information de séquence de la branche complémentaire inversée

$$f_{MET} = 1 - I_A^{CG} / <f_{CON}<I_A^A>$$

$I_T^{CG}$ désignant l'intensité du signal de thymine d'une cytosine en position CpG et $<I_T^T>$ l'intensité moyenne du signal de thymine aux positions de thymine ;
$I_A^{CG}$ désignant l'intensité du signal d'adénine d'une guanine en position CpG et $<I_A^A>$ l'intensité moyenne du signal d'adénine aux positions d'adénine ;
un degré de méthylation de 1 correspondant à une méthylation à 100 % et
pour calculer un degré de méthylation $f_{MET}$, les composants utilisés à cet effet du taux de conversion $f_{CON}$ et du facteur de réduction $f_N$ se rapportant à chaque fois à la même branche que le degré de méthylation déterminé.

**20.** Procédé selon la revendication 2, dans lequel l'alignement les unes par rapport aux autres d'une séquence de bases d'un seul type de base s'effectue sur une séquence de nucléotides qui connue seulement partiellement et contenant trois nucléotides différents, correspondant à cette séquence dans un ADN non converti, après avoir déterminé les écarts entre deux bases individuelles quelconques en employant des méthodes de probabilité à l'aide de procédés statistiques et de signal théorique.

Fig 1

G G G T G G [AA] T T G T G T G T G T T T G G T G G T T
G G G C G G [AA] t C G C G T G C G t T C G G C G G t T
           0%           0%   0%      0%       0%    0%

A

G G G   C G G A T C G C G T G C G T T C G G C G G T T
G G G   C G G A t C G C G T G C G t T C G G C G G t T
     95%        95% 99%     96%     97%   99%

B

Fig 2